(19) Europäisches Patentamt / European Patent Office / Office européen des brevets

(11) **EP 2 898 045 B1**

(12) **EUROPÄISCHE PATENTSCHRIFT**

(45) Veröffentlichungstag und Bekanntmachung des Hinweises auf die Patenterteilung:
**24.10.2018 Patentblatt 2018/43**

(21) Anmeldenummer: **13758756.4**

(22) Anmeldetag: **03.09.2013**

(51) Int Cl.:
***C07C 15/54*** (2006.01) ***C07C 17/26*** (2006.01)
***C07C 25/24*** (2006.01) ***C09K 19/08*** (2006.01)
***G02F 1/13*** (2006.01) ***H01Q 3/30*** (2006.01)
***C09K 19/18*** (2006.01) ***C09K 19/34*** (2006.01)
***C09K 19/30*** (2006.01) ***C09K 19/32*** (2006.01)

(86) Internationale Anmeldenummer:
**PCT/EP2013/002642**

(87) Internationale Veröffentlichungsnummer:
**WO 2014/044357 (27.03.2014 Gazette 2014/13)**

(54) **VERBINDUNGEN MIT EINER C-C-DREIFACHBINDUNG UND DEREN VERWENDUNG IN FLÜSSIGKRISTALLMISCHUNGEN**

COMPOUNDS HAVING A C-C TRIPLE BOND AND USE THEREOF IN LIQUID-CRYSTAL MIXTURES

COMPOSÉS COMPRENANT UNE TRIPLE LIAISON C-C ET LEUR UTILISATION DANS DES MÉLANGES DE CRISTAUX LIQUIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.09.2012 EP 12006651**

(43) Veröffentlichungstag der Anmeldung:
**29.07.2015 Patentblatt 2015/31**

(73) Patentinhaber: **Merck Patent GmbH**
**64293 Darmstadt (DE)**

(72) Erfinder:
- **BROCKE, Constanze**
  **64521 Gross-Gerau (DE)**
- **JASPER, Christian**
  **63500 Seligenstadt (DE)**
- **PAULUTH, Detlef**
  **64372 Ober-Ramstadt (DE)**
- **MANABE, Atsutaka**
  **64625 Bensheim (DE)**

(56) Entgegenhaltungen:
**WO-A1-2011/035863** **WO-A1-2012/095139**
**WO-A1-2012/097853** **WO-A1-2012/126564**
**DE-A1-102011 112 950** **JP-A- 2005 015 406**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 898 045 B1

## Beschreibung

Die vorliegende Erfindung betrifft Verbindungen mit mindestens einer C-C-Dreifachbindung innerhalb einer Kette von wenigstens 3 Ringsystemen, die eine neutrale dielektrische Anisotropie aufweisen, deren Verwendung für Hochfrequenzbauteile, flüssigkristalline Medien enthaltend die Verbindungen und diese Medien enthaltende Hochfrequenzbauteile, insbesondere Antennen, speziell für den Gigahertzbereich. Die flüssigkristallinen Medien dienen beispielsweise zur Phasenschiebung von Mikrowellen für abstimmbare 'phased-array' Antennen.

Flüssigkristalline Medien werden seit längerem in elektrooptischen Anzeigen (Liquid Crystal Displays - LCDs) genutzt, um Informationen anzuzeigen.

In neuerer Zeit werden flüssigkristalline Medien jedoch auch für die Verwendung in Komponenten, bzw. in Bauteilen, für die Mikrowellentechnik vorgeschlagen, wie z.B. in DE 10 2004 029 429 A und in JP 2005-120208 (A).

Eine technisch wertvolle Anwendung der flüssigkristallinen Medien in der Hochfrequenztechnik beruht auf ihrer Eigenschaft, dass sie sich durch eine variable Spannung in ihren dielektrischen Eigenschaften steuern lassen, besonders für den Gigahertzbereich. Somit lassen sich abstimmbare Antennen konstruieren, die keine beweglichen Teile beinhalten (A. Gaebler, A. Moessinger, F. Goelden, et al., "Liquid Crystal-Reconfigurable Antenna Concepts for Space Applications at Microwave and Millimeter Waves," International Journal of Antennas and Propagation, vol. 2009, Article ID 876989, 7 Seiten, 2009. doi:10.1155/2009/876989).

Die Druckschrift A. Penirschke, S. Müller, P. Scheele, C. Weil, M. Wittek, C. Hock und R. Jakoby: "Cavity Perturbation Method for Characterization of Liquid Crystals up to 35 GHz", 34th European Microwave Conference - Amsterdam, 545-548 beschreibt unter anderen die Eigenschaften der bekannten, flüssigkristallinen Einzelsubstanz K15 (Merck KGaA, Deutschland) bei einer Frequenz von 9 GHz.

DE 10 2004 029 429 A (vgl. oben) beschreibt die Anwendung von herkömmlichen Flüssigkristallmedien in der Mikrowellentechnik, unter anderem in Phasenschiebern. Dort werden bereits flüssigkristalline Medien bezüglich ihrer Eigenschaften im entsprechenden Frequenzbereich untersucht.

Verbindungen mit einer C-C-Dreifachbindung innerhalb einer Kette von 4 linear angeordneten Benzolringen werden in den Druckschriften JP 05-255151 A und WO 2009/125721 A1 offenbart. Die Verbindungen aus JP 05-255151 A sind teilweise mit Fluorsubstituenten versehen und finden als Komponente von flüssigkristallinen Medien Verwendung. Die in der zweiten Druckschrift offenbarten Verbindungen sind nur an den Molekülenden substituiert und dienen als Bestandteil von Dünnschichttransistoren.

Flüssigkristalline Verbindungen mit sehr hoher optischer Anisotropie und deutlich positiven Werten der dielektrischen Anisotropie sind bisher selten. Verbindungen dieser Art sind bestimmte Bistolane mit einer polaren Endgruppe, wie z.B. in den Druckschriften Shin-Tson Wu et al. Jpn. J. Appl. Phys. 1999, 38, 286-288, Shin-Tson Wu et al. Jpn. J. Appl. Phys. 2000, 39, 38-41, JP 10-45642 A und DE10120024 offenbart.

In WO 2009/125721 werden Verbindungen der Formel

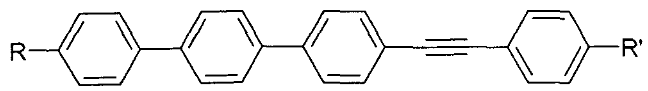

worin

R H und R' Alkyl mit 1 bis 13 C-Atomen oder $CF_3$,
R Methyl und R' H oder Methyl, oder
R und R' beide $CF_3$ bedeuten und
Verbindungen der Formel

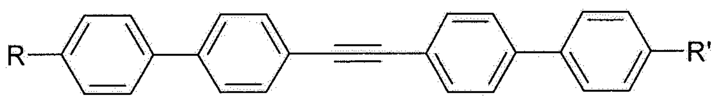

worin

R H und R' Methyl oder
R und R' beide Alkyl mit 1 bis 8 C-Atomen bedeuten
für die Anwendung in organischen Dünnfilmtransistoren vorgeschlagen.

In der bislang unveröffentlichten Patentanmeldung DE 10 2012 003 876.3 werden Verbindungen mit einer

terminalen polaren Gruppe z.B. der Formel

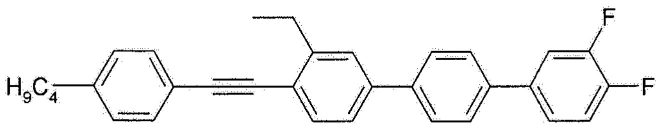

für die Anwendung in Bauteilen für die Hochfrquenztechnik vorgeschlagen.

Somit sind neue Komponenten für flüssigkristalline Medien mit verbesserten Eigenschaften erforderlich. Insbesondere müssen der Verlust im Mikrowellenbereich verringert und die Materialgüte (η) verbessert werden. Des weiteren finden Anwendungen in der Antennentechnik unter teilweise sich stark ändernden äußeren Rahmenbedingungen statt, wie z.B. starke Temperaturschwankungen. Besonders besteht der Bedarf, das Tieftemperaturverhalten der Bauteile zu verbessern.

Es besteht daher ein erheblicher Bedarf an flüssigkristallinen Medien mit geeigneten Eigenschaften für entsprechende praktische Anwendungen.

Überraschend wurde gefunden, dass die erfindungsgemäßen Verbindungen niedrige Schmelzpunkte und hohe Klärpunkte (Übergang der nematischen Phase in die isotrope Phase) aufweisen. Im flüssigkristallinen Bereich sind die Verbindungen überwiegend nematisch oder unterstützen die nematische Phase. Gleichzeitig liegt die optische Anisotropie (Δn) hohen Werten, wodurch sie sich z.B. für die Anwendung als Hochfrequenzmedium hervorragend eignen. Es wurde gefunden, dass mit den erfindungsgemäßen Verbindungen flüssigkristalline Medien mit einem breiten nematischen Phasenbereich und gleichzeitig hohen Werten für Δn und vorteilhaften Hochfrequenzeigenschaften verwirklicht werden können.

Die Erfindung betrifft Verbindungen der Formel I,

$$R^1\text{-}[A^1\text{-}Z^1]_m\text{-}A^2 \text{———}\equiv\text{———} A^3\text{-}[Z^2\text{-}A^4]_n\text{-}[Z^3\text{-}A^5]_o\text{-}R^2 \qquad \mathrm{I}$$

worin

einer von $A^2$ und $A^3$, bevorzugt $A^3$,

a) 1,4-Phenylen, worin ein oder mehrere, bevorzugt ein bis zwei CH-Gruppen durch N ersetzt sein können,
b) einen Rest der Formeln

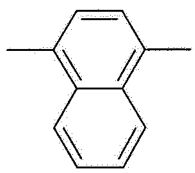 , 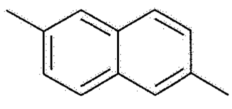 , 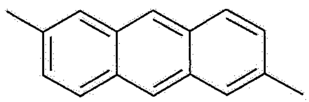 ,

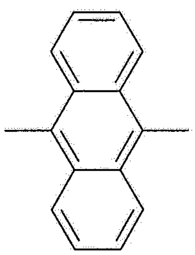 , 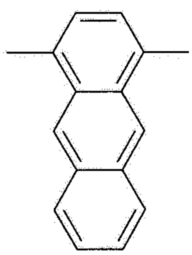 , 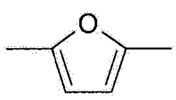 oder 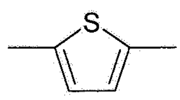 ,

c) trans-1,4-Cyclohexylen oder Cyclohexenylen, worin auch eine oder zwei nicht benachbarte $CH_2$-Gruppen durch -O- und/oder -S-ersetzt sein können, und worin H durch F ersetzt sein kann,
oder
d) einen Rest aus der Gruppe 1,4-Bicyclo[2.2.2]-octylen, Cyclobutan-1,3-diyl, Spiro[3.3]heptan-2,6-diyl, Thiophen-2,4-diyl, Furan-2,4-diyl,

und worin in den Gruppen a), b), c) und d)

optional ein oder mehrere H-Atome unabhängig gegen jeweils eine Gruppe Y ersetzt sind,

Y Br, Cl, F, CN, -NCS, -SCN, $SF_5$, $C_2$-$C_{10}$ Alkenyl, $C_1$-$C_{10}$ Alkoxy, $C_3$-$C_6$ Cycloalkyl, $C_3$-$C_6$ Cycloalkenyl oder eine ein- oder mehrfach fluorierte $C_1$-$C_{10}$ Alkyl- oder Alkoxygruppe, bevoruzgt Br, Cl, F, CN, -NCS, -SCN, $SF_5$, $OCF_3$ oder $CF_3$, besonders bevorzugt F,

a) 1,4-Phenylen, worin ein oder mehrere, bevorzugt ein bis zwei CH-Gruppen durch N ersetzt sein können,
b) einen Rest der Formeln

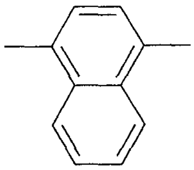
,
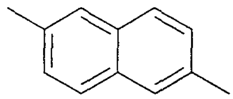
,
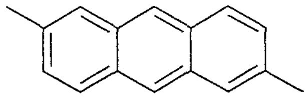
,

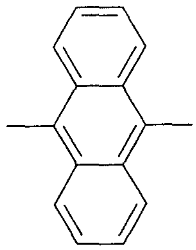
,
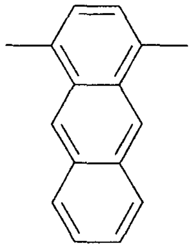
,
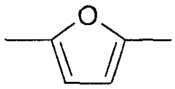
oder
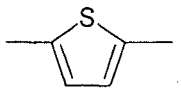
,

c) trans-1,4-Cyclohexylen oder Cyclohexenylen, worin auch eine oder zwei nicht benachbarte $CH_2$-Gruppen durch -O- und/oder -S-ersetzt sein können, und worin H durch F ersetzt sein kann,
oder
d) einen Rest aus der Gruppe 1,4-Bicyclo[2.2.2]-octylen, Cyclobutan-1,3-diyl, Spiro[3.3]heptan-2,6-diyl, Thiophen-2,4-diyl, Furan-2,4-diyl,

und worin in den Gruppen a), b), c) und d)
optional ein oder mehrere H-Atome unabhängig gegen jeweils eine Gruppe Y ersetzt sind,

Y Br, Cl, F, CN, -NCS, -SCN, $SF_5$, $C_2$-$C_{10}$ Alkenyl, $C_1$-$C_{10}$ Alkoxy, $C_3$-$C_6$ Cycloalkyl, $C_3$-$C_6$ Cycloalkenyl oder eine ein- oder mehrfach fluorierte $C_1$-$C_{10}$ Alkyl- oder Alkoxygruppe, bevoruzgt Br, Cl, F, CN, -NCS, -SCN, $SF_5$, $OCF_3$ oder $CF_3$, besonders bevorzugt F,

$R^1$ und $R^2$, unabhängig voneinander, einen halogenierten oder unsubstituierten Alkylrest mit 1 bis 15 C-Atomen, wobei in diesen

$R^1$ und $R^2$, einen geradkettigen Alkylrest mit 1 bis 15 C-Atomen, wobei in diesen Resten auch eine oder mehrere $CH_2$-Gruppen jeweils unabhängig voneinander durch -C≡C-, -CH=CH-, -(CO)O-, -O(CO)-, -(CO)-, -O- so ersetzt sein können, dass O-Atome nicht direkt miteinander verknüpft sind.

$R^3$ $C_1$-$C_{10}$ Alkyl, $C_2$-$C_{10}$ Alkenyl, $C_1$-$C_{10}$ Alkoxy, $C_3$-$C_6$ Cycloalkyl, $C_3$-$C_6$ Cycloalkenyl oder eine ein- oder mehrfach fluorierte $C_1$-$C_{10}$ Alkyl- oder Alkoxygruppe,

$Z^1$, $Z^2$ und $Z^3$, eine Einfachbindung, und

m, n und o, unabhängig 0 oder 1, wobei (m + n + o) 1, 2 oder 3 ist, bevorzugt 2 oder 3, besonders bevorzugt 2,

bedeuten.

Die erfindungsgemäßen Verbindungen besitzen einen vergleichsweise sehr niedrigen Schmelzpunkt, einen hohen Klärpunkt, eine hohe optische Anisotropie (Δn) und eine neutrale dielektrische Anisotropie. Die unerwünschte Rotation der Verbindungen ist eingeschränkt, so dass sie für die Anwendung im Gigahertzbereich besonders geeignet sind. Vorteilhaft ist der relativ geringe Verlustfaktor im Mikrowellenspektrum. Die Verbindungen besitzen allein oder in Mischung mit weiteren mesogenen Komponenten über einen breiten Temperaturbereich eine nematische Phase. Die

Gesamtheit dieser Eigenschaften machen sie besonders geeignet für die Verwendung in Bauteilen für die Hochfrequenztechnik, insbesondere in flüssigkristallinen Phasenschiebern. Erfindungsgemäße flüssigkristalline Medien besitzen die entsprechenden Eigenschaften.

Bevorzugte Verbindungen der Formel I sind durch die Auswahl eines oder mehrerer der folgenden Parameter gekennzeichnet:

Die Gruppen $A^1$, $A^2$, $A^3$, $A^4$ und $A^5$ umfassen bevorzugt Ringgruppen nach Definition a), b) oder c), besonders bevorzugt Ringgruppen nach Definition a) oder b), und ganz besonders bevorzugt nach Definition a).

Die Ringgruppen gemäß Definition a) haben bevorzugt die Teilstruktur

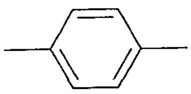
,
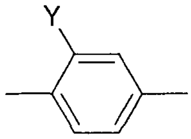
,
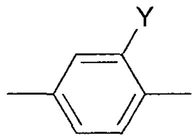
,
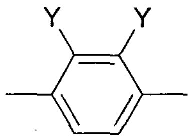

oder

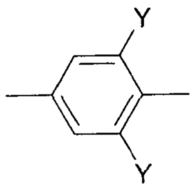

worin Y, bei jedem Auftreten unabhängig voneinander, die oben gegebene Bedeutung hat und bevorzugt Br, Cl, F, CN, -NCS, -SCN, $SF_5$, bedeutet.

Besonders bevorzugte Teilstrukturen "$-A^2-\equiv-A^3-$" sind dabei ausgewählt aus den folgenden Teilstrukturen:

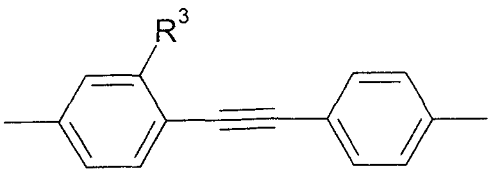
,
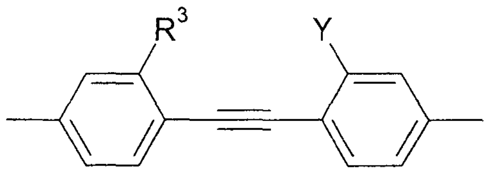
,

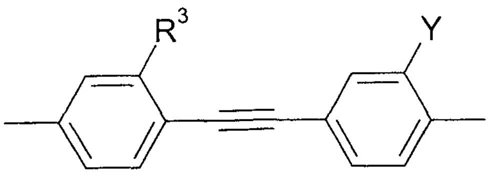
,
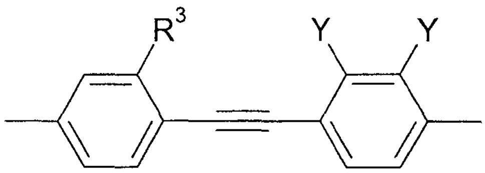
,

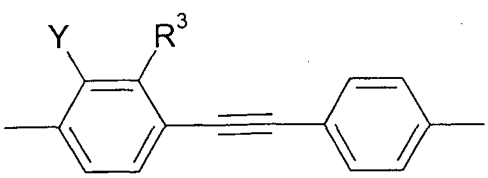
,
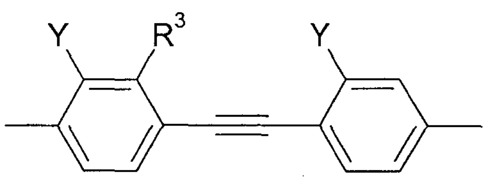
,

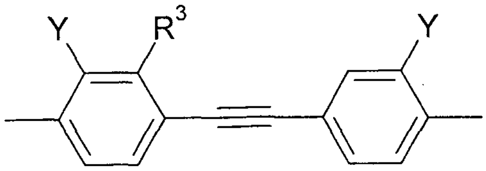,

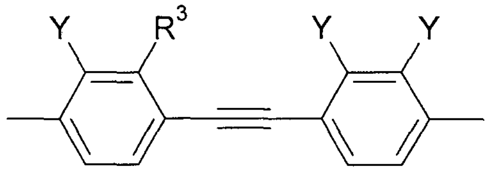,

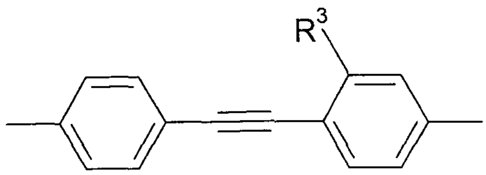,

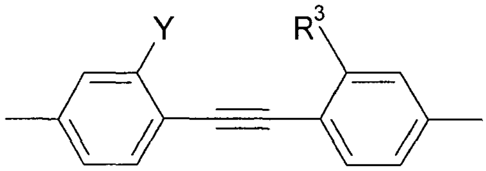,

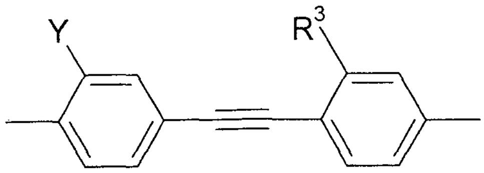,

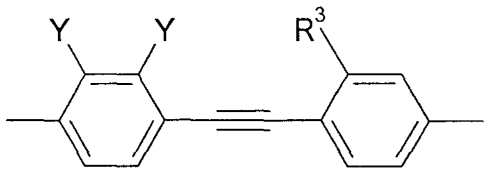,

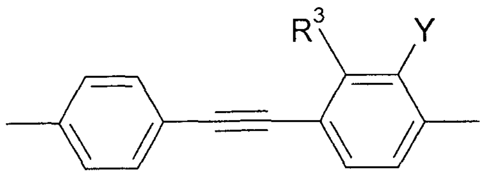,

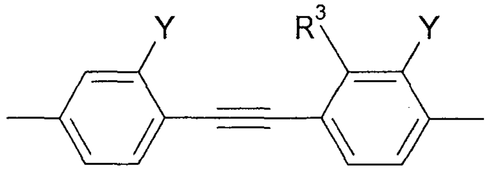,

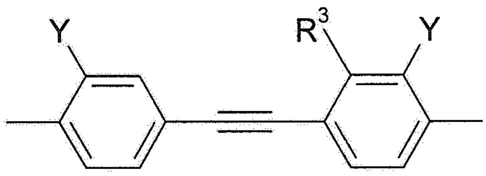

oder

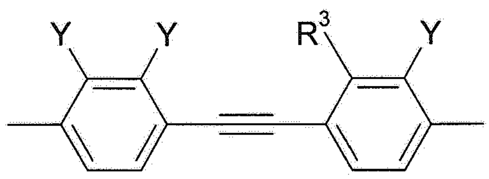,

bevorzugt

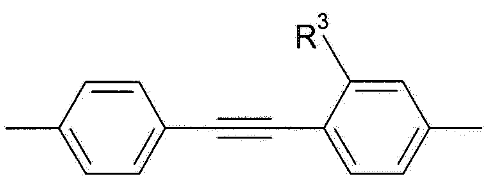

oder

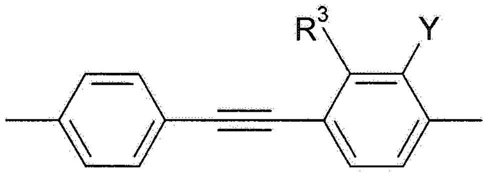

,

worin die Parameter die oben gegeben Bedeutungen haben.

Die Gruppe Y bedeutet bevorzugt F, Cl, Br, CN, $CF_3$, $OCF_3$, SCN, NCS, $SF_5$ oder einen halogenierten Alkyl- oder Alkoxyrest mit 1 bis 7 C-Atomen, wobei in diesem Rest auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)-, -O- oder -S- ersetzt sein können. Besonders bevorzugt bedeutet die Gruppe Y F, Cl, Br, -CN, -NCS, -SCN, -$SF_5$, fluoriertes Alkyl oder fluoriertes Alkoxy mit 1 bis 7 C-Atomen (z.B. $CF_3$ oder $OCF_3$) oder fluoriertes Alkenyl, fluoriertes Alkenyloxy (z. B. -$OCF=CF_2$) oder fluoriertes Alkoxyalkyl mit 2 bis 7 C-Atomen, ganz besonders bevorzugt fluoriertes Alkoxy, fluoriertes Alkenyloxy, F oder Cl.

Die Gruppe $R^3$ bedeutet bevorzugt Methyl, Ethyl, Propyl oder Cyclopropyl.

Bevorzugte Ausführungsformen der Erfindung werden daher durch die folgenden beispielhaften Strukturen repräsentiert:

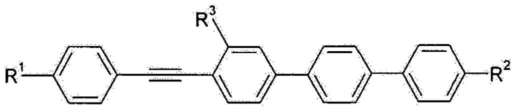

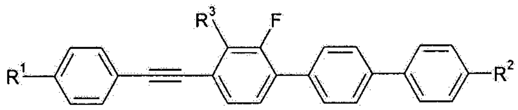

worin die Parameter die oben gegeben Bedeutungen haben und bevorzugt $R^1$ und $R^2$ einen Alkylrest, besonders bevorzugt n-Alkyl, besonders bevorzugt

$R^1$ einen Alkylrest mit 2 bis 7 C-Atomen, beispielsweise einen Propylrest oder einen Butyl-, Pentyl- oder Hexylrest,

$R^2$ einen Alkylrest mit 2 bis 7 C-Atomen, beispielsweise einen Propylrest oder einen Butyl-, Pentyl- oder Hexylrest,

$R^3$ einen Alkylrest mit 1 bis 7 C-Atomen, einen Alkenylrest mit 2 bis 7 C-Atomen, einen Cycloalkylrest mit 3 bis 6 C-Atomen oder einen Cycloalkenylrest mit 4 bis 6 C-Atomen, beispielsweise Ethyl, Propyl, Butyl, Pentyl, Hexyl, Cyclopropyl, Cyclopentyl, Cyclohexyl oder Cyclopentenyl, insbesondere Ethyl, Propyl oder Cyclopropyl,

bedeutet.

Die Verbindungen der Formel I werden nach an sich bekannten Methoden dargestellt, wie sie in der Literatur (z. B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

Typische Verbindungen der Formel I können vorteilhaft wie aus den folgenden beispielhaften Synthese-Schemata (Schemata 1 bis 3) ersichtlich hergestellt werden, worin die Parameter die oben gegeben Bedeutungen, inklusive der entsprechenden bevorzugten Bedeutungen, haben, sofern nicht anders angegeben.

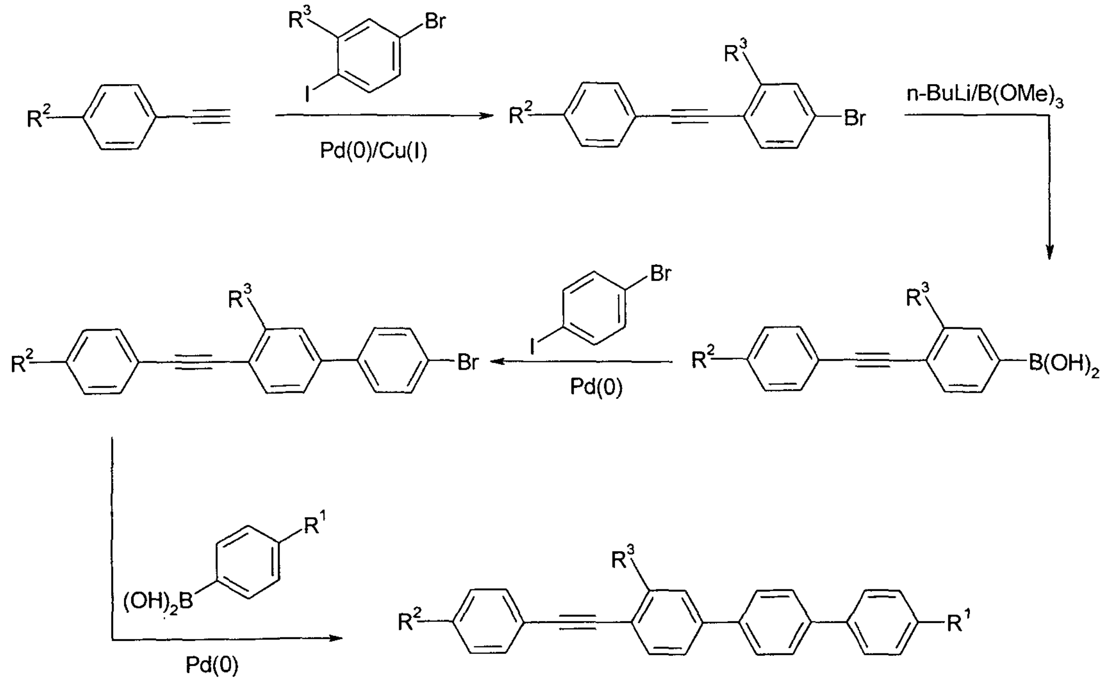


Schema 1: Syntheseschema zur Darstellung von Verbindungen der Formel I worin m = 0 und (n + o) = 2 bedeuten.

$R^1$, $R^2$ und $R^3$ in Schemata 1 bis 3 haben die Bedeutung von $R^1$, $R^2$ und $R^3$ wie vor- und nachstehend, insbesondere wie für Formel I definiert. In Reaktionsschema 1 wird die Synthese von bestimmten Verbindungen wiedergegeben. Die Phenylreste "$R^1$-Phenyl" lassen sich dabei auf beliebige Reste "$R^1$-($A^1$-$Z^1$)$_m$-$A^2$-" gemäß Formel I verallgemeinern. Die übrigen Ringe können ebenso gemäß Formel I in Art und Substitution variiert werden.

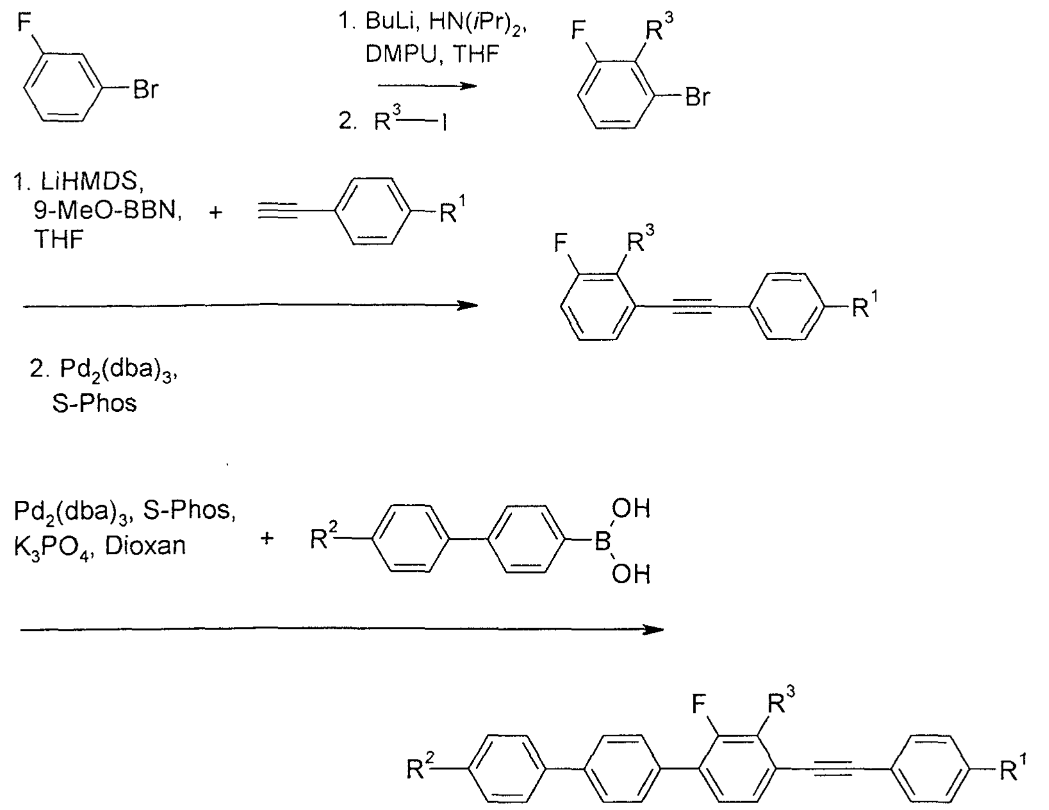


Schema 2: Syntheseschema zur Darstellung von Verbindungen der Formel I worin m = 0 und (n + o) = 2 bedeuten.

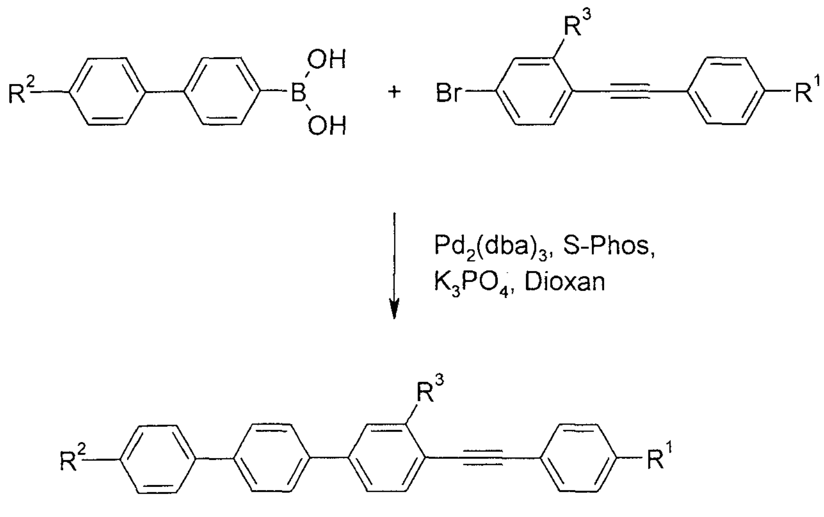


Schema 3: Syntheseschema zur Darstellung von Verbindungen der Formel I worin m = 0 und (n + o) = 2 bedeuten.

Die flüssigkristallinen Medien gemäß der vorliegenden Erfindung enthalten eine oder mehrere Verbindungen der Formel I und optional mindestens eine weitere, vorzugsweise mesogene Verbindung. Das Flüssigkristallmedium enthält daher bevorzugt zwei oder mehr Verbindungen, die vorzugsweise flüssigkristallin sind. Bevorzugte Medien umfassen die bevorzugten Verbindungen der Formel I.

Weitere Komponenten der flüssigkristallinen Medien sind vorzugsweise ausgewählt aus den Verbindungen der Formel II:

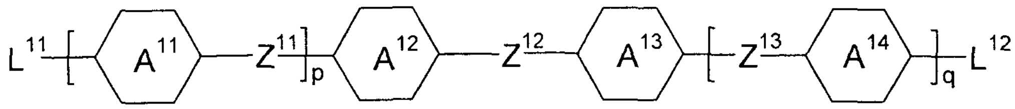 II

worin

| | |
|---|---|
| $L^{11}$ | $R^{11}$ oder $X^{11}$, |
| $L^{12}$ | $R^{12}$ oder $X^{12}$, |
| $R^{11}$ und $R^{12}$ | unabhängig voneinander unfluoriertes Alkyl oder unfluoriertes Alkoxy mit 1 bis 17, bevorzugt mit 3 bis 10, C-Atomen oder unfluoriertes Alkenyl, unfluoriertes Alkinyl, unfluoriertes Alkenyloxy, oder unfluoriertes Alkoxyalkyl mit 2 bis 15, bevorzugt 3 bis 10, C-Atomen, vorzugsweise Alkyl oder unfluoriertes Alkenyl bedeuten, |
| $X^{11}$ und $X^{12}$ | unabhängig voneinander F, Cl, Br, -CN, -NCS, -SCN, -$SF_5$, fluoriertes Alkyl oder fluoriertes Alkoxy mit 1 bis 7 C-Atomen oder fluoriertes Alkenyl, fluoriertes Alkenyloxy oder fluoriertes Alkoxyalkyl mit 2 bis 7 C-Atomen, vorzugsweise fluoriertes Alkoxy, fluoriertes Alkenyloxy, F oder Cl bedeuten, |
| p, q | unabhängig 0 oder 1, |
| $Z^{11}$ bis $Z^{13}$ | unabhängig voneinander *trans*- -CH=CH-, *trans*- -CF=CF-, -C≡C- oder eine Einfachbindung bedeuten, und |

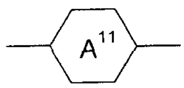

bis

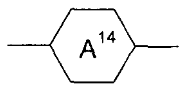

unabhängig voneinander

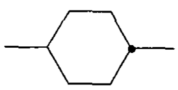 , 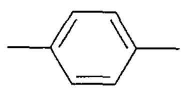 , 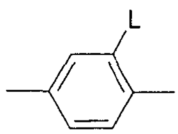 ,

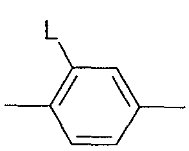 , 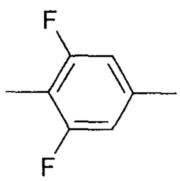 , 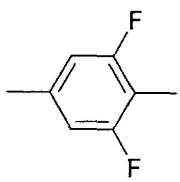 ,

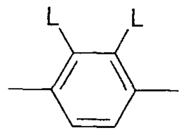

,

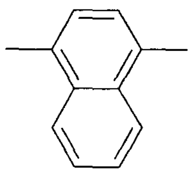

,

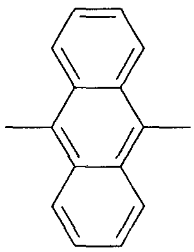

oder

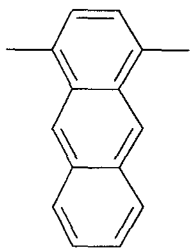

L, bei jedem Auftreten unabhängig voneinander, verzweigtes oder unverzweigtes Alkyl, Alkenyl oder Alkinyl mit 1 bis 12 C-Atomen, worin unabhängig voneinander auch eine oder mehrere "-$CH_2$-"-Gruppen durch O ersetzt sein können, $C_3$-$C_6$ Cycloalkyl, $C_3$-$C_6$ Cycloalkenyl, fluoriertes Alkyl oder Alkenyl, fluoriertes Alkyloxy oder Alkenyloxy, F, Cl, Br, CN, NCS, SCN oder $SF_5$,

bedeuten.

In einer bevorzugten Ausführungsform der vorliegenden Erfindung enthalten die flüssigkristallinen Medien eine oder mehrere Verbindungen der Formel I und eine oder mehrere Verbindungen der Formel II.

Bevorzugt enthalten die flüssigkristallinen Medien gemäß der vorliegenden Anmeldung insgesamt 5 bis 95 %, bevorzugt 10 bis 90 % und besonders bevorzugt 15 bis 80 %, an Verbindungen der Formel I.

Vorzugsweise enthalten die flüssigkristallinen Medien gemäß der vorliegenden Erfindung Verbindungen ausgewählt aus der Gruppe der Verbindungen der Formeln I und II, stärker bevorzugt bestehen sie überwiegend, noch stärker bevorzugt bestehen sie im Wesentlichen und ganz bevorzugt bestehen sie vollständig daraus.

In dieser Anmeldung bedeutet "enthalten" im Zusammenhang mit Zusammensetzungen, dass die betreffende Entität, d.h. das Medium oder die Komponente, die angegebene Komponente oder Komponenten oder Verbindung oder Verbindungen enthält, vorzugsweise in einer Gesamtkonzentration von 10 % oder mehr und ganz bevorzugt von 20 % oder mehr.

"Überwiegend bestehen" bedeutet in diesem Zusammenhang, dass die betreffende Entität 55 % oder mehr, vorzugsweise 60 % oder mehr und ganz bevorzugt 70 % oder mehr der angegebenen Komponente oder Komponenten oder Verbindung oder Verbindungen enthält.

"Im Wesentlichen" bestehen bedeutet in diesem Zusammenhang, dass die betreffende Entität 80 % oder mehr, vorzugsweise 90 % oder mehr und ganz bevorzugt 95 % oder mehr der angegebenen Komponente oder Komponenten oder Verbindung oder Verbindungen enthält.

"Vollständig bestehen" bedeutet in diesem Zusammenhang, dass die betreffende Entität 98 % oder mehr, vorzugsweise 99 % oder mehr und ganz bevorzugt 100,0 % der angegebenen Komponente oder Komponenten oder Verbindung oder Verbindungen enthält.

Bevorzugt enthalten die flüssigkristallinen Medien gemäß der vorliegenden Anmeldung insgesamt 10 bis 100 %, bevorzugt 20 bis 95 % und besonders bevorzugt 25 bis 90 %, an Verbindungen der Formel I und II.

Gemäß der vorliegenden Erfindung werden die Verbindungen der Formel II vorzugsweise in einer Gesamtkonzentration von 10 % bis 90 %, stärker bevorzugt von 15 % bis 85 %, noch stärker bevorzugt von 25 % bis 80 % und ganz bevorzugt von 30 % bis 75 % der Gesamtmischung verwendet.

Die flüssigkristallinen Medien können darüber hinaus weitere Zusätze wie Stabilisatoren, chirale Dotierstoffe und Nanopartikel enthalten. Die einzelnen, zugesetzten Verbindungen werden in Konzentrationen von 0,005 bis 6 %, vorzugsweise von 0,1 bis 3 %, eingesetzt. Die Gesamtkonzentration dieser weiteren Bestandteile liegt im Bereich von 0 % bis 10 %, vorzugsweise 0,1 % bis 6 %, bezogen auf die Gesamtmischung. Dabei werden jedoch die Konzentrationsangaben der übrigen Bestandteile der Flüssigkristallmischungen, also der flüssigkristallinen oder mesogenen Verbindungen, ohne Berücksichtigung der Konzentration dieser Zusatzstoffe angegeben.

Vorzugsweise enthalten die flüssigkristallinen Medien 0 bis 10 Gew.-%, insbesondere 0,01 bis 5 Gew.-% und besonders bevorzugt 0,1 bis 3 Gew.-% an Stabilisatoren. Vorzugsweise enthalten die Medien einen oder mehrere Stabilisatoren ausgewählt aus 2,6-Di-tert-butylphenolen, 2,2,6,6-Tetramethylpiperidinen oder 2-Benzotriazol-2-yl-phenolen. Diese Hilfsstoffe sind dem Fachmann bekannt und kommerziell erhältlich, z. B. als Lichtschutzmittel.

Eine Ausführungsform der Erfindung ist daher auch ein Verfahren zur Herstellung eines Flüssigkristallmediums, das dadurch gekennzeichnet ist, dass eine oder mehrere Verbindungen der Formel I mit einer oder mehreren weiteren

Verbindungen und optional mit einem oder mehreren Additiven gemischt wird. Die weiteren Verbindungen sind vorzugsweise ausgewählt aus den Verbindungen der Formel II, wie oben angegeben, und optional einer oder mehreren weiteren Verbindungen.

Ein weiterer Gegenstand der vorliegenden Anmeldung ist ein Verfahren zu Herstellung von 2-Alkyl-1-brom-3-fluorbenzolen, dadurch gekennzeichnet, dass 1-Brom-3-fluorbenzol zunächst mit LDA *ortho*-metalliert wird und dann *in situ* mit dem entsprechenden Alkyliodid alkyliert wird.

In der vorliegenden Anmeldung beschreibt der Ausdruck dielektrisch positiv Verbindungen oder Komponenten mit $\Delta\varepsilon > 3,0$, dielektrisch neutral mit $-1,5 \leq \Delta\varepsilon \leq 3,0$ und dielektrisch negativ mit $\Delta\varepsilon < -1,5$. Die dielektrische Anisotropie der jeweiligen Verbindung wird aus den Ergebnissen einer Lösung von 10 % der jeweiligen einzelnen Verbindung in einer nematischen Host-Mischung bestimmt. Wenn die Löslichkeit der jeweiligen Verbindung in der Host-Mischung weniger als 10 % beträgt, wird die Konzentration auf 5 % reduziert. Die Kapazitäten der Testmischungen werden sowohl in einer Zelle mit homöotroper als auch mit homogener Orientierung bestimmt. Die Schichtdicke beträgt bei beiden Zelltypen ca. 20 μm. Die angelegte Spannung ist eine Rechteckwelle mit einer Frequenz von 1 kHz und einem Effektivwert von typischerweise 0,5 V bis 1,0 V, wird jedoch stets so ausgewählt, dass sie unterhalb der kapazitiven Schwelle für die jeweilige Testmischung liegt.

$\Delta\varepsilon$ ist als $(\varepsilon_{\parallel} - \varepsilon_{\perp})$ definiert, während $\varepsilon_{Durchschnitt}$ $(\varepsilon_{\parallel} + 2\ \varepsilon_{\perp})$ / 3 ist.

Als Host-Mischung wird für dielektrisch positive Verbindungen die Mischung ZLI-4792 und für dielektrisch neutrale sowie für dielektrisch negative Verbindungen die Mischung ZLI-3086 verwendet, beide von Merck KGaA, Deutschland. Die absoluten Werte der dielektrischen Konstanten der Verbindungen werden aus der Änderung der jeweiligen Werte der Host-Mischung bei Zugabe der interessierenden Verbindungen bestimmt. Die Werte werden auf eine Konzentration der interessierenden Verbindungen von 100 % extrapoliert.

Komponenten, die bei der Messtemperatur von 20°C eine nematische Phase aufweisen, werden als solche gemessen, alle anderen werden wie Verbindungen behandelt.

Der Ausdruck Schwellenspannung bezeichnet in der vorliegenden Anmeldung die optische Schwelle und ist für 10 % relativen Kontrast ($V_{10}$) angegeben, der Ausdruck Sättigungsspannung bezeichnet die optische Sättigung und ist für 90 % relativen Kontrast ($V_{90}$) angegeben, in beiden Fällen, soweit nicht ausdrücklich etwas anderes angegeben ist. Die kapazitive Schwellenspannung ($V_0$), auch Freedericks-Schwelle $V_{Fr}$ genannt, wird nur verwendet, wenn dies ausdrücklich genannt ist.

Die in dieser Anmeldung angegebenen Parameterbereiche schließen sämtlich die Grenzwerte ein, wenn nicht ausdrücklich etwas anderes angegeben ist.

Die unterschiedlichen für verschiedene Bereiche von Eigenschaften angegebenen oberen und unteren Grenzwerte ergeben in Kombination miteinander zusätzliche bevorzugte Bereiche.

In der gesamten Anmeldung gelten, wenn nicht ausdrücklich anders angegeben, die folgenden Bedingungen und Definitionen. Alle Konzentrationen sind in Massenprozent angegeben und beziehen sich jeweils auf die Gesamtmischung, alle Temperaturen und alle Temperaturunterschiede sind in Grad Celsius bzw. Differenzgrad angegeben. Alle für Flüssigkristalle typischen physikalischen Eigenschaften werden nach "Merck Liquid Crystals, Physical Properties of Liquid Crystals", Stand Nov. 1997, Merck KGaA, Deutschland, bestimmt und sind für eine Temperatur von 20°C aufgeführt, wenn nicht ausdrücklich anders angegeben. Die optische Anisotropie ($\Delta n$) wird bei einer Wellenlänge von 589,3 nm bestimmt. Die dielektrische Anisotropie ($\Delta\varepsilon$) wird bei einer Frequenz von 1 kHz bestimmt. Die Schwellenspannungen sowie alle anderen elektrooptischen Eigenschaften werden mit bei Merck KGaA, Deutschland, hergestellten Testzellen bestimmt. Die Testzellen für die Bestimmung von $\Delta\varepsilon$ besitzen eine Schichtdicke von circa 20 μm. Bei der Elektrode handelt es sich um eine kreisförmige ITO-Elektrode mit einer Fläche von 1,13 $cm^2$ und einem Schutzring. Die Ausrichtungsschichten sind SE-1211 von Nissan Chemicals, Japan, für homöotrope Ausrichtung ($\varepsilon_{\parallel}$) und Polyimid AL-1054 von Japan Synthetic Rubber, Japan, für homogene Ausrichtung ($\varepsilon_{\perp}$). Die Bestimmung der Kapazitäten erfolgt mit einem Frequenzgang-Analysegerät Solatron 1260 unter Verwendung einer Sinuswelle mit einer Spannung von 0,3 $V_{rms}$. Als Licht wird bei den elektrooptischen Messungen weißes Licht verwendet. Dabei wird ein Aufbau mit einem im Handel erhältlichen Gerät DMS der Fa. Autronic-Melchers, Germany verwendet. Die charakteristischen Spannungen werden unter senkrechter Beobachtung bestimmt. Die Schwellenspannung ($V_{10}$), "Mittgrau-Spannung" ($V_{50}$) und Sättigungsspannung ($V_{90}$) werden für 10 %, 50 % bzw. 90 % relativen Kontrast bestimmt.

Die flüssigkristallinen Medien werden bezüglich ihrer Eigenschaften im Frequenzbereich der Mikrowellen untersucht wie in A. Penirschke et al. "Cavity Perturbation Method for Characterization of Liquid Crystals up to 35GHz", 34th European Microwave Conference - Amsterdam, S. 545-548 beschreiben. Vergleiche hierzu auch A. Gaebler et al. "Direct Simulation of Material Permittivities ...", 12MTC 2009 - International Instrumentation and Measurement Technology Conference, Singapur, 2009 (IEEE), S. 463-467 und DE 10 2004 029 429 A, in der ebenfalls detailliert ein Messverfahren beschrieben wird.

Der Flüssigkristall wird in eine Kapillare aus Polytetrafluorethylen (PTFE) oder Quarzglas gefüllt. Die Kapillare hat einen inneren Radius von 180 μm und einen äußeren Radius von 350 μm. Die effektive Länge beträgt 2,0 cm. Die gefüllte Kapillare wird in die Mitte der zylindrischen Kavität mit einer Resonanzfrequenz von 19 GHz eingebracht. Diese

Kavität hat eine Länge von 11,5 mm und einen Radius von 6 mm. Daraufhin wird das Eingangssignal ("source") angelegt und das Ergebnis des Ausgangssignals mit einem kommerziellen Netzwerkanalysator ("vector network analyzer") aufgenommen. Für andere Frequenzen werden die Abmessungen der Kavität entsprechend angepasst.

Aus der Änderung der Resonanzfrequenz und des Q-Faktors, zwischen der Messung mit der mit dem Flüssigkristall gefüllten Kapillare und der Messung ohne der mit dem Flüssigkristall gefüllten Kapillare, wird die dielektrische Konstante und der Verlustwinkel bei der entsprechenden Zielfrequenz mittels der Gleichungen 10 und 11 der zuvor genannten Druckschrift A. Penirschke et al., 34th European Microwave Conference - Amsterdam, S. 545-548 bestimmt, wie dort beschrieben.

Die Werte für die Komponenten der Eigenschaften senkrecht bzw. and parallel zum Direktor des Flüssigkristalls werden durch Orientierung des Flüssigkristalls in einem Magnetfeld erhalten. Dazu wird das Magnetfeld eines Permanentmagneten verwendet. Die Stärke des Magnetfelds beträgt 0,35 Tesla. Die Orientierung des Magneten wird entsprechend eingestellt und dann entsprechend um 90° gedreht.

Die dielektrische Anisotropie im $\mu$-Wellenbereich ist definiert als

$$\Delta\varepsilon_r \equiv (\varepsilon_{r,||} - \varepsilon_{r,\perp}) \ .$$

Die Modulierbarkeit bzw. Steuerbarkeit ("tuneability", $\tau$) ist definiert als

$$\tau \equiv (\Delta\varepsilon_r / \varepsilon_{r,||}) \ .$$

Die Materialgüte ($\eta$) ist definiert als

$$\eta \equiv (\tau / \tan \delta_{\varepsilon\, r,Max.}) \ ,$$

mit dem maximalen dielektrischen Verlustfaktor tan $\delta_{\varepsilon r,Max}$:

$$\tan \delta_{\varepsilon\, r,Max.} \equiv Max. \{ \tan \delta_{\varepsilon\, r,\perp} ; \tan \delta_{\varepsilon\, r,||} \}$$

der aus dem Maximalwert der gemessenen Werte für tan$\delta_\varepsilon$, hervorgeht.

Die Materialgüte ($\eta$) der bevorzugten Flüssigkristallmaterialien beträgt 6 oder mehr, bevorzugt 7 oder mehr, bevorzugt 10 oder mehr, bevorzugt 15 oder mehr, besonders bevorzugt 25 oder mehr und ganz besonders bevorzugt 30 oder mehr.

Die bevorzugten Flüssigkristallmaterialien haben in den entsprechenden Bauteilen Phasenschiebergüten von 15°/dB oder mehr, bevorzugt von 20°/dB oder mehr, bevorzugt von 30°/dB oder mehr, bevorzugt von 40°/dB oder mehr, bevorzugt von 50°/dB oder mehr, besonders bevorzugt von 80°/dB oder mehr und ganz besonders bevorzugt von 100°/dB oder mehr.

Die erfindungsgemäßen Flüssigkristallmedien weisen bevorzugt nematische Phasen von jeweils mindestens von -20°C bis 80°C, bevorzugt von -30°C bis 85°C und ganz besonders bevorzugt von -40°C bis 100°C auf. Insbesondere bevorzugt reicht die Phase bis 120°C oder mehr, bevorzugt bis 140°C oder mehr und ganz besonders bevorzugt bis 180°C oder mehr. Hierbei bedeutet der Begriff eine nematische Phase aufweisen einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, mit einer Schichtdicke von 5 $\mu$m, für mindestens 100 Stunden überprüft. Bei hohen Temperaturen wird der Klärpunkt nach üblichen Methoden in Kapillaren gemessen.

Die Flüssigkristallmedien gemäß der vorliegenden Erfindung weisen bevorzugt einen Klärpunkt von 90°C oder mehr, stärker bevorzugt von 100°C oder mehr, noch stärker bevorzugt von 120°C oder mehr, besonders bevorzugt von 150°C oder mehr und ganz besonders bevorzugt von 170°C oder mehr, auf.

Das $\Delta\varepsilon$ des Flüssigkristallmediums gemäß der Erfindung bei 1 kHz und 20°C beträgt vorzugsweise 1 oder mehr, stärker bevorzugt 2 oder mehr und ganz bevorzugt 3 oder mehr.

Das $\Delta n$ der Flüssigkristallmedien gemäß der vorliegenden Erfindung liegt bei 589 nm ($Na^D$) und 20°C vorzugsweise im Bereich von 0,20 oder mehr bis 0,90 oder weniger, stärker bevorzugt im Bereich von 0,25 oder mehr bis 0,90 oder weniger, noch stärker bevorzugt im Bereich von 0,30 oder mehr bis 0,85 oder weniger und ganz besonders bevorzugt im Bereich von 0,35 oder mehr bis 0,80 oder weniger.

In einer bevorzugten Ausführungsform der vorliegenden Anmeldung beträgt das $\Delta n$ der Flüssigkristallmedien gemäß der vorliegenden Erfindung vorzugsweise 0,50 oder mehr, stärker bevorzugt 0,55 oder mehr.

Ferner sind die erfindungsgemäßen Flüssigkristallmedien durch hohe Anisotropien im Mikrowellenbereich gekennzeichnet. Die Doppelbrechung beträgt z.B. bei ca. 8,3 GHz bevorzugt 0,14 oder mehr, besonders bevorzugt 0,15 oder mehr, besonders bevorzugt 0,20 oder mehr, besonders bevorzugt 0,25 oder mehr und ganz besonders bevorzugt 0,30 oder mehr. Außerdem beträgt die Doppelbrechung bevorzugt 0,80 oder weniger.

Die eingesetzten Flüssigkristalle sind entweder Einzelsubstanzen oder Mischungen. Bevorzugt weisen sie eine nematische Phase auf.

In der vorliegenden Anmeldung, bedeutet, wenn nicht ausdrücklich anders angegeben, der Begriff Verbindungen sowohl eine Verbindung, als auch mehrere Verbindungen.

Bevorzugte Bauelemente, die ein Flüssigkristallmedium oder wenigstens eine Verbindung gemäß der Erfindung enthalten, sind Phasenschieber, Varaktoren, Antennenarrays (z. B. für Funk, Mobilfunk, Radio, Mikrowellen/Radar und sonstige Datenübertragung), 'matching circuit adaptive filters' und andere. Bevorzugt sind Bauteile für die Hochfrequenztechnik, wie oben definiert. Bevorzugt sind außerdem durch unterschiedliche angelegte elektrische Spannungen modulierbare Bauteile. Ganz besonders bevorzugte Bauelemente sind abstimmbare Phasenschieber. In bevorzugten Ausführungsformen werden mehrere Phasenschieber funktionell verbunden, wodurch beispielsweise ein phasengesteuerte Gruppenantenne, in der Regel als 'phased array' Antenne bezeichnet, resultiert. Eine Gruppenantenne nutzt die Phasenverschiebung der in einer Matrix angeordneten Sende- oder Empfangselemente, um durch Interferenz eine Bündelung zu erzielen. Aus einer reihen- oder gitterförmigen parallelen Anordnung von Phasenschiebern lässt sich ein sog. 'phased array' aufbauen, das als abstimmbare oder passive Sende- oder Empfangsantenne für Hochfrequenzen (z. B. Gigahertzbereich) dienen kann. Erfindungsgemäße 'phased array'-Antennen besitzen einen sehr breiten nutzbaren Empfangskegel.

Bevorzugte Anwendungen sind Radarinstallationen und Datenübertragungsgeräte auf bemannten oder unbemannten Fahrzeugen aus dem Bereich Automobil, Schifffahrt, Flugzeuge, Raumfahrt und Satellitentechnik.

Zur Herstellung geeigneter Bauteile für die Hochfrequenztechnik, insbesondere geeigneter Phasenschieber, wird typischerweise ein erfindungsgemäßes flüssigkristallines Medium in rechteckige Kavitäten von weniger als 1 mm Dicke, mehreren Millimetern Breite und mehreren Zentimetern Länge eingebracht. Die Kavitäten besitzen entlang zweier langer Seiten angebrachte, gegenüberliegende Elektroden. Solche Anordnungen sind dem Fachmann vertraut. Durch Anlegen einer variablen Spannung können beim Betreiben der Antenne die dielektrischen Eigenschaften des flüssigkristallinen Mediums abgestimmt werden, um verschiedene Frequenzen oder Richtungen einer Antenne einzustellen.

Der Ausdruck "Halogen" oder "halogeniert" steht für F, Cl, Br und I, besonders für F und Cl und insbesondere für F. Ein halogenierter Alylrest bedeutet daher bevorzugt einen chlorierten oder fluorierten Alkylrest.

Der Ausdruck "Alkyl" umfasst vorzugsweise geradkettige und verzweigte Alkylgruppen mit 1 bis 15 Kohlenstoffatomen, insbesondere die geradkettigen Gruppen Methyl, Ethyl, Propyl, Butyl, Pentyl, Hexyl und Heptyl. Gruppen mit 2 bis 10 Kohlenstoffatomen sind im Allgemeinen bevorzugt.

Der Ausdruck "Alkenyl" umfasst vorzugsweise geradkettige und verzweigte Alkenylgruppen mit 2 bis 15 Kohlenstoffatomen, insbesondere die geradkettigen Gruppen. Besonders bevorzugte Alkenylgruppen sind $C_2$ bis $C_7$-1E-Alkenyl, $C_4$ bis $C_7$-3E-Alkenyl, $C_5$ bis $C_7$-4-Alkenyl, $C_6$ bis $C_7$-5-Alkenyl und $C_7$-6-Alkenyl, insbesondere $C_2$ bis $C_7$-1E-Alkenyl, $C_4$ bis $C_7$-3E-Alkenyl und $C_5$ bis $C_7$-4-Alkenyl. Beispiele weiterer bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Alkoxy" umfasst vorzugsweise geradkettige Reste der Formel $C_nH_{2n+1}$-O-, worin n 1 bis 10 bedeuten. Vorzugsweise ist n 1 bis 6. Bevorzugte Alkoxygruppen sind beispielsweise Methoxy, Ethoxy, n-Propoxy, n-Butoxy, n-Pentoxy, n-Hexoxy, n-Heptoxy, n-Octoxy, n-Nonoxy, n-Decoxy.

Der Ausdruck "Oxaalkyl" bzw. "Alkoxyalkyl" umfasst vorzugsweise geradkettige Reste der Formel $C_nH_{2n+1}$-O-$(CH_2)_m$, worin n und m jeweils unabhängig voneinander 1 bis 10 bedeuten. Vorzugsweise ist n 1 und m 1 bis 6.

Der Ausdruck "fluorierter Alkylrest" umfasst vorzugsweise ein- oder mehrfach fluorierte Reste. Perfluorierte Reste sind eingeschlossen. Bevorzugt sind $CF_3$, $CH_2CF_3$, $CH_2CHF_2$, $CHF_2$, $CH_2F$, $CHFCF_3$ und $CF_2CHFCF_3$, besonders bevorzugt $CF_3$.

Der Ausdruck "fluorierter Alkoxyrest" umfasst ein- oder mehrfach fluorierte Reste. Perfluorierte Reste sind bevorzugt. Besonders bevorzugt ist der Rest $OCF_3$.

Der Ausdruck "Alk(en/in)ylgruppen, worin ein oder mehrere "-$CH_2$-"-Gruppen durch -O- ersetzt sein können" bezieht sich vorzugsweise auf solche Gruppen, worin eine nicht-terminale $CH_2$-Gruppe ersetzt wird. OH-Gruppen sind in der allgemeinen Bedeutung mit umfasst.

Der Ausdruck "substituiertes Cycloalkyl" umfasst ein- oder mehrfach durch Alkyl substituiertes Cycloalkyl, insbesondere Alkyl mit 1 bis 8 Kohlenstoffatomen.

Der Ausdruck "substituiertes Phenyl" umfasst ein- oder mehrfach durch eine Gruppe wie $R^1$ definiert substitu-

iertes Phenyl, insbesondere durch F, Cl, Alkyl oder Alkoxy substituiertes Phenyl.

In der vorliegenden Anmeldung bedeutet Hochfrequenztechnik Anwendungen mit Frequenzen im Bereich von 1 MHz bis 10 THz, bevorzugt von 1 GHz bis 3 THz, stärker bevorzugt 2 GHz bis 1 THz, insbesondere bevorzugt von 5 bis 300 GHz. Die Anwendung liegt bevorzugt im Mikrowellenspektrum oder angrenzenden, für die Nachrichtenübertragung geeigneten Bereichen, in denen 'phased array'-Module in Sende- und Empfangsantennen zum Einsatz kommen können.

Die erfindungsgemäßen Flüssigkristallmedien bestehen aus einer oder mehreren Verbindungen, vorzugsweise aus 2 bis 30, stärker bevorzugt aus 3 bis 20 und ganz bevorzugt aus 3 bis 16 Verbindungen. Diese Verbindungen werden auf herkömmliche Weise gemischt. In der Regel wird die gewünschte Menge der in der geringeren Menge verwendeten Verbindung in der in der größeren Menge verwendeten Verbindung gelöst. Liegt die Temperatur über dem Klärpunkt der in der höheren Konzentration verwendeten Verbindung, ist die Vervollständigung des Lösungsvorgangs besonders leicht zu beobachten. Es ist jedoch auch möglich, die Medien auf anderen üblichen Wegen, beispielsweise unter Verwendung von so genannten Vormischungen, bei denen es sich z.B. um homologe oder eutektische Mischungen von Verbindungen handeln kann, oder unter Verwendung von so genannten "Multi-Bottle"-Systemen, deren Bestandteile selbst gebrauchsfertige Mischungen sind, herzustellen. Alle Temperaturen, wie z.B. der Schmelzpunkt T(K,N) bzw. T(K,S), der Übergang von der smektischen (S) zur nematischen (N) Phase T(S,N) und der Klärpunkt T (N,I) der Flüssigkristalle sind in Grad Celsius angegeben. Alle Temperaturdifferenzen sind in Differenzgraden angegeben.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste $C_nH_{2n+1}$ und $C_mH_{2m+1}$ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt getrennt von Akronym für den Grundkörper mit einem Strich ein Code für die Substituenten $R^{1*}$, $R^{2*}$, $L^{1*}$ und $L^{2*}$:

| Code für $R^{1*}$, $R^{2*}$, $L^{1*}$, $L^{2*}$, $L^{3*}$ | $R^{1*}$ | $R^{2*}$ | $L^{1*}$ | $L^{2*}$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | F | H |
| nN.F.F | $C_nH_{2n+1}$ | CN | F | F |
| nF | $C_nH_{2n+1}$ | F | H | H |
| nCl | $C_nH_{2n+1}$ | Cl | H | H |
| nOF | $OC_nH_{2n+1}$ | F | H | H |
| nF.F | $C_nH_{2n+1}$ | F | F | H |
| nF.F.F | $C_nH_{2n+1}$ | F | F | F |
| $nOCF_3$ | $C_nH_{2n+1}$ | $OCF_3$ | H | H |
| $nOCF_3.F$ | $C_nH_{2n+1}$ | $OCF_3$ | F | H |
| n-Vm | $C_nH_{2n+1}$ | $-CH=CH-C_mH_{2m+1}$ | H | H |
| nV-Vm | $C_nH_{2n+1}-CH=CH-$ | $-CH=CH-C_mH_{2m+1}$ | H | H |

Geeignete Mischungskomponenten finden sich in den Tabellen A und B.

Tabelle A

| | |
|---|---|
| 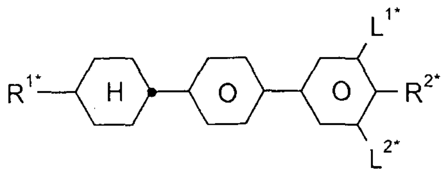 | 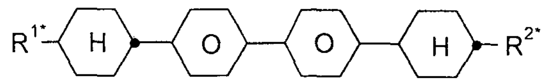 |
| **BCH** | **CBC** |
| 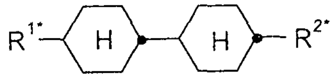 | 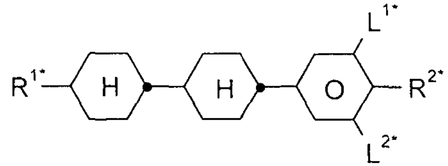 |
| **CCH** | **CCP** |
| 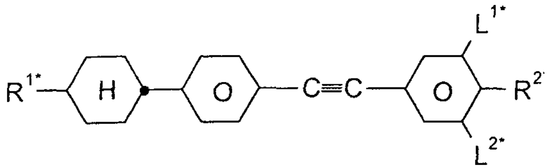 | 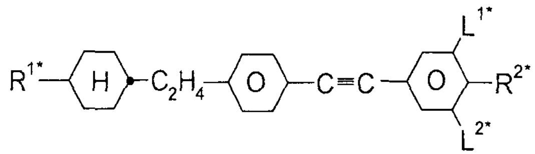 |
| **CPTP** | **CEPTP** |
| 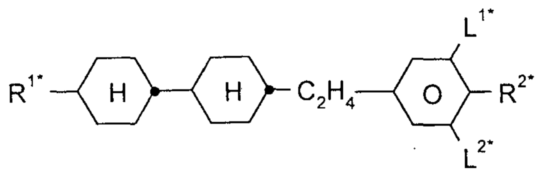 | 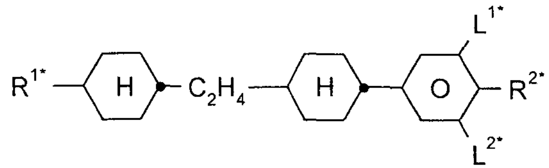 |
| **ECCP** | **CECP** |
| 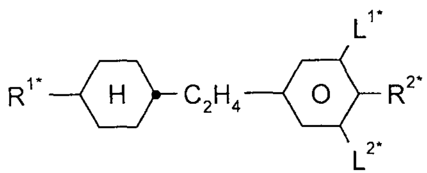 | 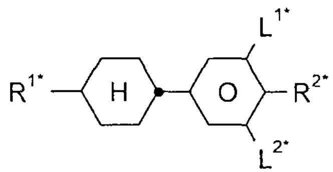 |
| **EPCH** | **PCH** |

(fortgesetzt)

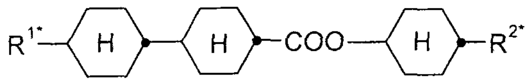

CH

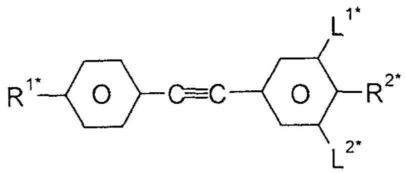

PTP

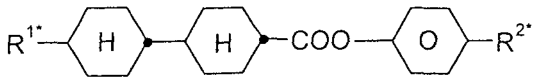

CP

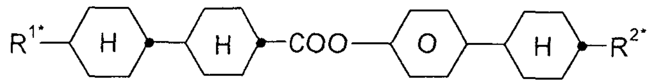

CCPC

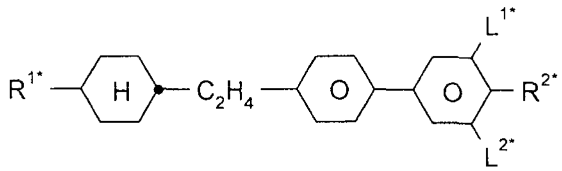

BECH

**Tabelle B**

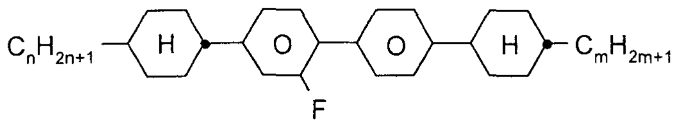

**CBC-nmF**

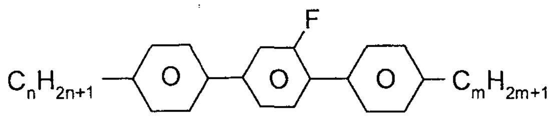

**PGP-n-m**

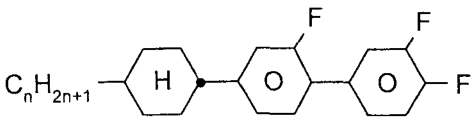

**CGG-n-F**

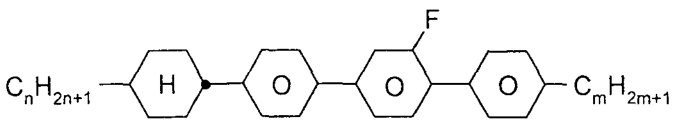

**CPGP-n-m**

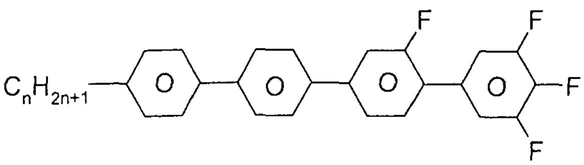

**PPGU-n-F**

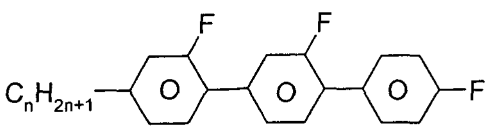

**GGP-n-F**

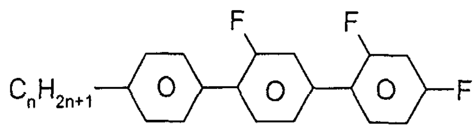

**PGIGI-n-F**

**PPGU-n-F**

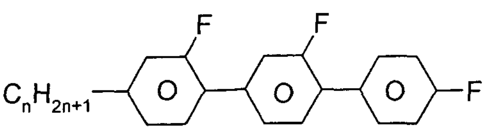

**GGP-n-F**

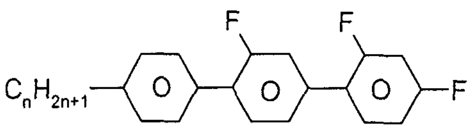

**PGIGI-n-F**

Die folgenden Beispiele erläutern die vorliegende Erfindung, ohne sie in irgendeiner Weise zu beschränken.

Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

In der vorliegenden Anmeldung, bedeutet, wenn nicht ausdrücklich anders angegeben, die Pluralform eines Begriffs sowohl die Singularform als auch die Pluralform, und umgekehrt. Weitere Kombinationen der Ausführungsformen

und Varianten der Erfindung gemäß der Beschreibung ergeben sich auch aus den angefügten Ansprüchen.

Verwendete Abkürzungen:

| | |
|---|---|
| MTB | Methyl-*tert*-butylether, |
| RT | Raum- bzw. Umgebungstemperatur (ca. 20 °C), |
| DMPU | Dimethyltetrahydro-2(1*H*)-pyrimidinon, |
| THF | Tetrahydrofuran, |
| LiHMDS | Lithium-bis(trimethylsilyl)amid, |
| LDA | Lithiumdiisopropylamid, |
| 9-MeO-BBN | 9-Methoxy-9-borabicyclo[3.3.1]nonan, |
| $Pd_2(dpa)_3$ | Tris(dibenzylidenaceton)-dipalladium und |
| S-Phos | 2-Dicyclohexylphosphino-2',6'-dimethoxybiphenyl. |

**Beispiele**

Die eingesetzten Acetylene und Boronsäuren sind kommerziell erhältlich oder können in Analogie zu bekannten Synthesen hergestellt werden, die dem Fachmann bekannt sind. Die Reste "$C_4H_9$" stehen für unverzweigte n-Butylreste. Entsprechendes gilt für $C_3H_7$, $C_5H_{11}$, $C_6H_{13}$ u.s.w..

**<u>Synthesebeispiel 1: Synthese von 4-Butyl-4"-(4-butyl-phenylethinyl)-2"-fluor-3"-propyl-[1,1';4',1"]terphenyl (1)</u>**

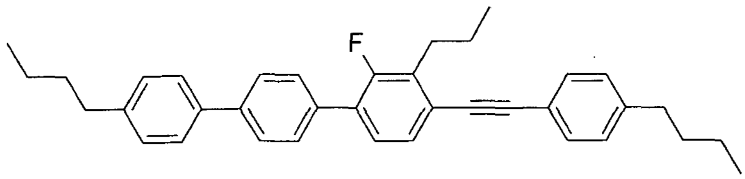

(**1**)

Die Synthese von 4-Butyl-4"-(4-butyl-phenylethinyl)-2"-fluor-3"-propyl-[1,1';4',1"]terphenyl (1) erfolgt ausgehend von 1-Brom-3-fluorbenzol (**2**) (CAS 1073-06-9).

**1.1 Synthese von 1-Brom-3-fluor-2-propylbenzol (3)**

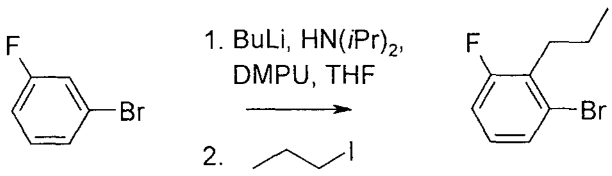


(**2**) (**3**)

Eine Lösung von Diisopropylamin (46,4 mL, 0,33 mol) und 1,3-Dimethyltetrahydro-2(1*H*)-pyrimidinon (42,3 g, 0,33 mol) in THF (750 mL) wird bei -70°C tropfenweise mit *n*-Butyllithium (206,5 mL, 1,6 M in Hexan, 0,33 mol) versetzt und 30 min bei 0°C gerührt. Das Reaktionsgemisch wird auf -75°C abgekühlt, und bei dieser Temperatur wird eine Lösung aus 1-Brom-3-fluorbenzol (**2**) (54,6 g, 0,31 mol) in THF (250 mL) langsam zugetropft und 1 h nachgerührt. Anschließend wird bei -70°C 1-Iodpropan (30,0 mL, 0,31 mol) zugetropft und das Reaktionsgemisch 16 h unter Rühren auf Raumtemperatur erwärmen lassen. Zur Aufarbeitung wird mit Aus WO 2012/097853 A1 sind Verbindungen der folgenden Struktur

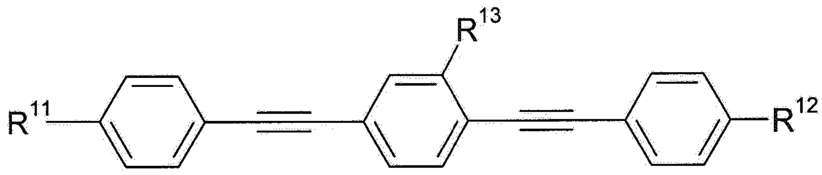

worin die Reste $R^{11}$ bis $R^{13}$ beispielsweise Alkyl bedeuten,als für die Anwendung in der Hochfrequenztechnik vorteilhaft beschrieben. Derivate dieser Struktur und flüssikristalline Medien für die Hochfrequenztechnik, die solche Verbindungen enthalten, sind aus WO 2012/097853 A1 bekannt. Auch DE 10 2011 1112950 A1 lehrt ähnliche Verbindungen, darunter z.B. solche, die einen zusätzlichen aromatischen Ring oder eine weitere Dreifachbindung enthalten:

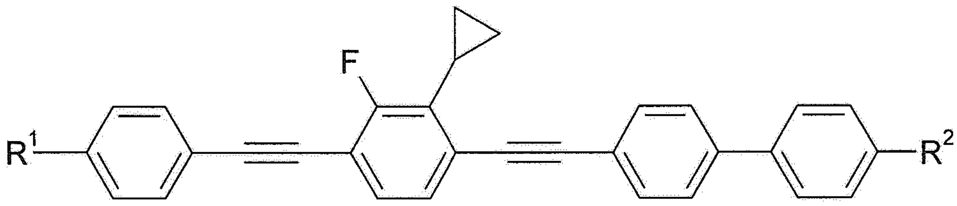

Naphthalinderivate dieses Typs, ebenfalls zur Verwendung in der Hochfrequenztechnik, sind aus WO 2012/095139 A1 bekannt.

Die bisher bekannten Zusammensetzungen oder Einzelverbindungen sind jedoch in der Regel mit Nachteilen behaftet. Die meisten von ihnen führen, neben anderen Mängeln, zu unvorteilhaft hohen Verlusten und/oder unzureichenden Phasenverschiebungen bzw. zu geringer Materialgüte. Während beispielsweise manche Einzelverbindungen keine günstigen flüssigkristallinen Phasen und sehr hohe Schmelzpunkte aufweisen, mangelt es wiederum anderen Substanzen an genügend hohen Werten für $\Delta n$ und $\Delta\varepsilon$.

Für die Anwendung in der Hochfrequenztechnik werden flüssigkristalline Medien mit besonderen, bislang eher ungewöhnlichen, ungebräuchlichen Eigenschaften, bzw. Kombinationen von Eigenschaften benötigt.

**1.3 Synthese von 1-(4-Butyl-phenylethinyl)-3-fluor-4-iod-2-propyl-benzol (6)**

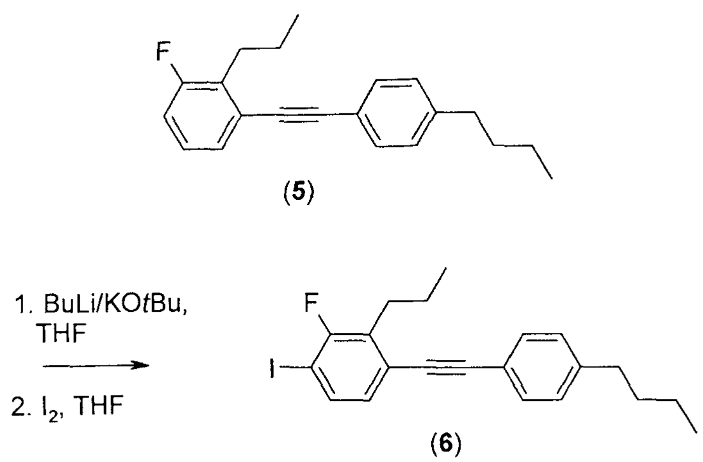


(5)

(6)

Ein Gemisch aus 1-(4-Butyl-phenylethinyl)-3-fluor-2-propylbenzol (5) (43,5 g, 0,14 mol) und Kalium-*tert*-butanolat (17,3 g, 0,15 mol) in THF (500 mL) wird bei -90°C tropfenweise mit n-Butyllithium (97,0 mL, 1,6 M in Hexan, 0,15 mol) versetzt und 45 min bei dieser Temperatur gerührt. Danach wird eine Lösung aus Iod (39,3 g, 0,15 mol) in THF (250 mL) zugetropft, und das Reaktionsgemisch wird weitere 30 min bei -90°C gerührt und anschließend über 3 h unter Rühren auf -20°C aufgetaut. Zur Aufarbeitung wird mit dest. Wasser hydrolysiert, bis zur Entfärbung mit Natriumhydrogensulfit versetzt und die organische Phase abgetrennt. Die wässrige Phase wird mit Methyl-*tert*-butylether extrahiert, und die vereinigten organischen Phasen werden mit dest. Wasser gewaschen, über Natriumsulfat getrocknet und im Vakuum eingeengt. Der Rückstand wird durch Filtration über Kieselgel (100% Heptan) gereinigt und 1-(4-Butyl-phenylethinyl)-3-fluor-4-iod-2-propylbenzol (**6**) als gelbes Öl isoliert.

**1.4 Synthese von 4-Butyl-4"-(4-butyl-phenylethinyl)-2"-fluor-3"-propyl-[1,1';4',1"]terphenyl (1)**

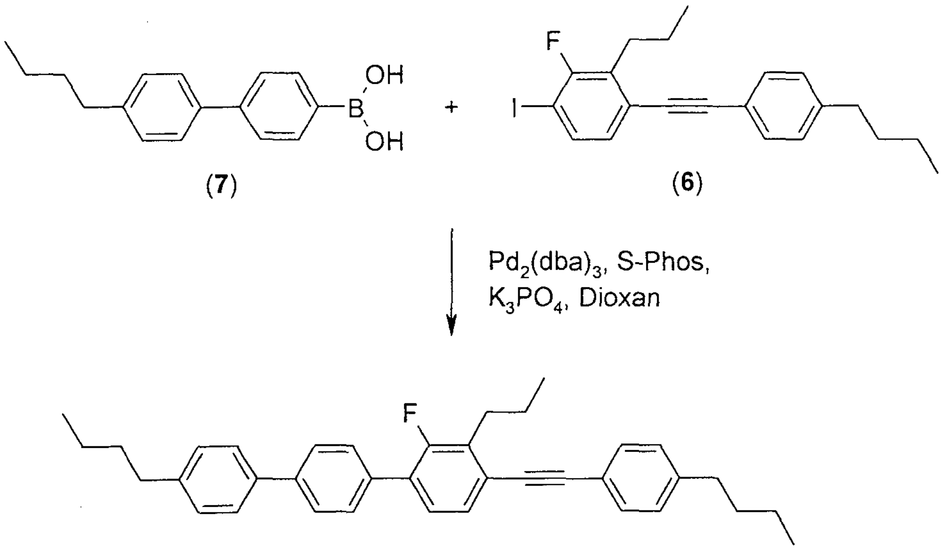


(**7**) (**6**)

(**1**)

Eine Mischung aus 1-(4-Butyl-phenylethinyl)-3-fluor-4-iod-2-propylbenzol (6) (2,8 g, 10,6 mmol), 4'-Butyl-biphenyl-4-boronsäure (7) (CAS 145413-17-8) (5,0 g, 10,6 mmol), Tris(dibenzylidenaceton)dipalladium (196 mg, 0,2 mmol), 2-Dicyclohexylphosphino-2',6'-dimethoxybiphenyl (357 mg, 0,8 mmol) und Kaliumphosphat (12,2 g, 53,0 mmol) in 1,4-Dioxan (100 mL) wird mit 3 Tropfen dest. Wasser versetzt und 16 h bei 100°C gerührt. Die Aufarbeitung erfolgt durch Zugabe von Methyl-*tert*-butylether und dest. Wasser. Nach Abtrennung der organischen Phase wird die wässrige Phase erneut mit Methyl-*tert*-butylether extrahiert. Die vereinigten organischen Phasen werden mit dest. Wasser und Natriumchlorid-Lösung gewaschen, über Natriumsulfat getrocknet und im Vakuum eingeengt. Der Rückstand wird über Kieselgel filtriert (Heptan/Chlorbutan 8:2) und das Rohprodukt durch Umkristallisation aus Ethanol/Toluol (2x) aufgereinigt. 4-Butyl-4"-(4-butyl-phenylethinyl)-2"-fluor-3"-propyl-[1,1';4',1"]terphenyl (**1**) wird in Form von farblosen Kristallen isoliert.

**MS (EI):** m/z = 502 ($M^+$), 473 ($[M - Ethyl]^+$), 459 ($[M - Propyl]^+$), 430 ($[M - Propyl - Ethyl]^+$), 387 ($[M - 2 Propyl - Ethyl]^+$).

K 107°C N 217°C I;

$\Delta\varepsilon$ = 0,8;
$\Delta n$ = 0,362 und
$\gamma_1$ = 5.490 Pa·s.

**<u>Synthesebeispiel 2:</u>**

**<u>Synthese von 4-Butyl-4"-(4-butyl-phenylethinyl)-3"-ethyl-[1,1';4',1"]terphenyl) (9)</u>**

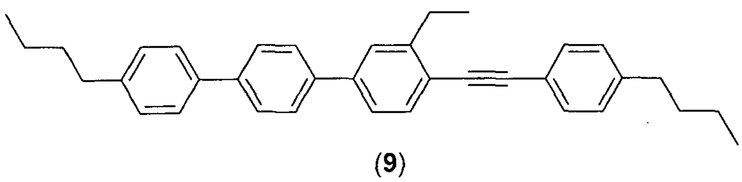

(**9**)

Die Synthese von 4-Butyl-4"-(4-butyl-phenylethinyl)-3"-ethyl-[1,1';4',1"]terphenyl) (**9**) erfolgt ausgehend von 4'-Butyl-biphenyl-4-boronsäure (**7**) (CAS 145413-17-8) und 4-Brom-1-(4-butyl-phenylethinyl)-2-ethylbenzol (**8**) (CAS 1375922-99-8).

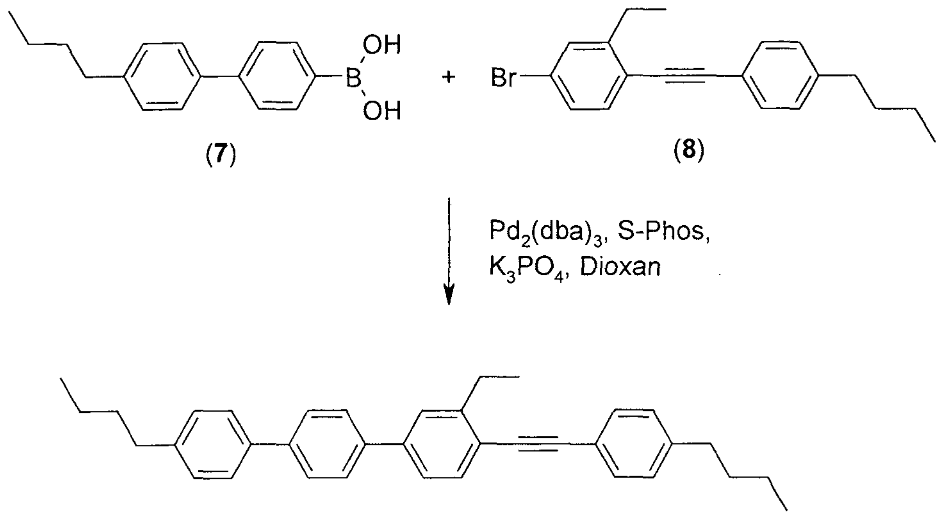


(**7**) (**8**)

(**9**)

Eine Mischung aus 4-Brom-1-(4-butyl-phenylethinyl)-2-ethylbenzol (**9**) (CAS 1375922-99-8) (5,0 g, 14,3 mmol), 4'-Butyl-biphenyl-4-boronsäure (**7**) (CAS 145413-17-8) (3,7 g, 14,3 mmol), Tris(dibenzylidenaceton)-dipalladium (263 mg, 0,3 mmol), 2-Dicyclohexylphosphino-2',6'-dimethoxybiphenyl (480 mg, 1,1 mmol) und Kaliumphosphat (16,4 g, 71.3 mmol) in 1,4-Dioxan (100 mL) wird mit 3 Tropfen dest. Wasser versetzt und 4 h bei 100°C gerührt. Die Aufarbeitung erfolgt durch Zugabe von Methyl-*tert*-butylether und dest. Wasser. Nach Abtrennung der organischen Phase wird die wässrige Phase erneut mit Methyl-*tert*-butylether extrahiert. Die vereinigten organischen Phasen werden mit dest. Wasser und Natriumchlorid-Lösung gewaschen, über Natriumsulfat getrocknet und im Vakuum eingeengt. Der Rückstand wird über Kieselgel filtriert (100% Heptan) und das Rohprodukt durch Umkristallisation aus Ethanol/Toluol (2x), Heptan und Isopropanol/Toluol aufgereinigt. 4-Butyl-4"-(4-butyl-phenylethinyl)-3"-ethyl-[1,1';4',1"]terphenyl) (**9**) wird in Form von farblosen Kristallen isoliert.

**MS (EI):** m/z = 470 ($M^+$), 455 ($[M - Methyl]^+$), 427 ($[M - Propyl]^+$), 412 ($[M - Propyl - Methyl]^+$), 369 ($[M - 2 Propyl - Methyl]^+$).

K 132°C N 260°C I;

$\Delta\varepsilon$ = 1,8;
$\Delta n$ = 0,413 und
$\gamma_1$ = 5.250 Pa·s.

**Mischungsbeispiel 1**

Es wird ein Flüssigkristallmedium M-1 mit der Zusammensetzung und den Eigenschaften wie in der folgenden Tabelle angegeben hergestellt. Die Verbindung (**1**) stammt aus dem Synthesebeispiel 1.

| Zusammensetzung | | | Physikalische Eigenschaften | | | |
|---|---|---|---|---|---|---|
| Verbindung | | | T(N,I) | = | 96 | °C |
| Nr. | Abkürzung | | | | | |
| 1 | BCH-3F.F | 10,8 % | | | | |
| 2 | BCH-5F.F | 9,0 % | $\Delta n$ (20°C, 589,3 nm) | = | 0,124 | |
| 3 | ECCP-30CF3 | 4,5 % | | | | |
| 4 | ECCP-50CF3 | 4,5 % | | | | |
| 5 | CBC-33F | 1,8 % | $\Delta\varepsilon$ (20°C, 1 kHz) | = | 4,9 | |
| 6 | CBC-53F | 1,8 % | | | | |
| 7 | CBC-55F | 1,8 % | $\gamma_1$ (20°C) | = | 166 | mPa · s |

(fortgesetzt)

| Zusammensetzung | | | Physikalische Eigenschaften |
|---|---|---|---|
| 8 | PCH-6F | 7,2 % | |
| 9 | PCH-7F | 5,4 % | |
| 10 | CCP-20CF3 | 7,2 % | |
| 11 | CCP-30CF3 | 10,8 % | |
| 12 | CCP-40CF3 | 6,3 % | |
| 13 | CCP-50CF3 | 9,9 % | |
| 14 | PCH-5F | 9,0 % | |
| 15 | (1) | 10,0 % | |
| Σ | | 100,0 % | |

Diese Mischung wird bevorzugt für Anwendungen im Mikrowellenbereich, insbesondere für Phasenschieber z.B. für 'phased array' Antennen verwendet.

Zum Vergleich wird ein Medium C ohne die Komponente (**1**) aus den Verbindungen Nr. 1-14 des Mediums M-1 hergestellt, wobei die Verbindungen Nr. 1-14 in den gleichen relativen Mengen enthalten sind.

**Mischungsbeispiel 2**

Es wird, wie bei Mischungsbeispiel 1, ein Flüssigkristallmedium M-2 mit der Zusammensetzung von M-1 hergestellt, mit dem Unterschied, dass für M-2 anstelle der Verbindung (1) die Verbindung (**9**) aus Synthesebeispiel 2 eingesetzt wird.

Auch diese Mischung wird bevorzugt für Anwendungen im Mikrowellenbereich, insbesondere für Phasenschieber z.B. für 'phased array' Antennen verwendet.

Die Ergebnisse zu den Mischungsbeispielen finden sich in der nachfolgenden Tabelle.

Tabelle: Eigenschaften der Mischungen M-1 und M-2, und C (zum Vergleich) bei 19 GHz (20°C)

| Mischung | $\varepsilon_{r,\parallel}$ | $\varepsilon_{r,\perp}$ | $\tau$ | tan $\delta_{\varepsilon, r,\parallel}$ | tan $\delta_{\varepsilon, r,\perp}$ | $\eta$ |
|---|---|---|---|---|---|---|
| M-1 | 2,56 | 2,25 | 0,121 | 0,0040 | 0,0107 | 11,3 |
| M-2 | 2,57 | 2,25 | 0,124 | 0,0039 | 0,0110 | 11,2 |
| C | 2,56 | 2,29 | 0,107 | 0,0049 | 0,0126 | 8,5 |

Die Steuerbarkeit $\tau$ und die Materialgüte $\eta$ sind für die beiden erfindungsgemäßen Mischungen M-1 und M-2 gegenüber denen den Vergleichsmischungen C deutlich verbessert.

**Patentansprüche**

**1.** Verbindungen der Formel I

$$R^1\text{-}[A^1\text{-}Z^1]_m\text{-}A^2 \equiv A^3\text{-}[Z^2\text{-}A^4]_n\text{-}[Z^3\text{-}A^5]_o\text{-}R^2 \qquad \text{I}$$

worin

einer von $A^2$ und $A^3$

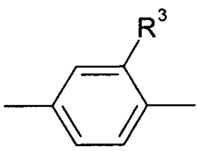 , 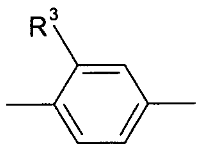 , 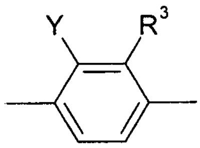 , 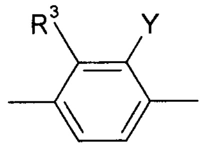 ,

oder

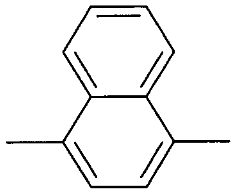 ,

der andere von $A^2$ und $A^3$, $A^1$, $A^4$ und $A^5$, unabhängig voneinander

a) 1,4-Phenylen, worin ein oder mehrere CH-Gruppen durch N ersetzt sein können,
b) einen Rest der Formeln

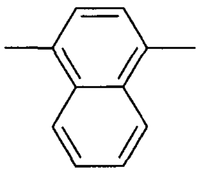 , 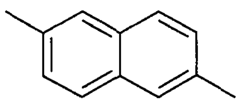 , 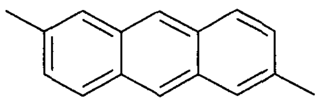 ,

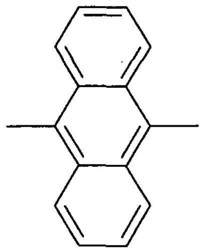 , 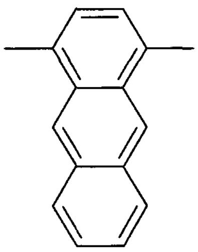 , 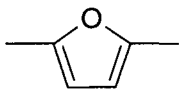 oder 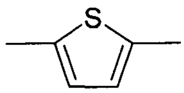 ,

c) trans-1,4-Cyclohexylen oder Cyclohexenylen, worin auch eine oder zwei nicht benachbarte $CH_2$-Gruppen durch -O- und/oder -S-ersetzt sein können,
oder
d) einen Rest aus der Gruppe 1,4-Bicyclo(2,2,2)-octylen, Cyclobutan-1,3-diyl, Spiro[3.3]heptan-2,6-diyl, Thiophen-2,5-diyl, Furan-2,5-diyl,

und worin in den Gruppen a), b), c) und d) optional ein oder mehrere H-Atome unabhängig gegen jeweils eine Gruppe Y ersetzt sind,
Y Br, Cl, F, CN, -NCS, -SCN, $SF_5$, $C_2$-$C_{10}$ Alkenyl, $C_1$-$C_{10}$ Alkoxy, $C_3$-$C_6$ Cycloalkyl, $C_3$-$C_6$ Cycloalkenyl oder eine ein- oder mehrfach fluorierte $C_1$-$C_{10}$ Alkyl- oder Alkoxygruppe,
$R^1$ und $R^2$, einen geradkettigen Alkylrest mit 1 bis 15 C-Atomen, wobei in diesen Resten auch eine oder mehrere $CH_2$-Gruppen jeweils unabhängig voneinander durch -C≡C-, -CH=CH-, -(CO)O-, -O(CO)-, -(CO)-, -O- so ersetzt sein können, dass O-Atome nicht direkt miteinander verknüpft sind.,
$R^3$ $C_1$-$C_{10}$ Alkyl, $C_2$-$C_{10}$ Alkenyl, $C_1$-$C_{10}$ Alkoxy, $C_3$-$C_6$ Cycloalkyl, $C_3$-$C_6$ Cycloalkenyl oder eine ein- oder mehrfach fluorierte $C_1$-$C_{10}$ Alkyl- oder Alkoxygruppe,
$Z^1$, $Z^2$ und $Z^3$, eine Einfachbindung, und
m, n und o, unabhängig voneinander, 0 oder 1, wobei (m + n + o) 2 oder 3 ist,

bedeuten.

**2.** Verbindungen nach Anspruch 1, **dadurch gekennzeichnet, dass** die Teilformel "-$A^2$-≡-$A^3$-" der Formel I ausgewählt ist aus der Gruppe der folgenden Formeln

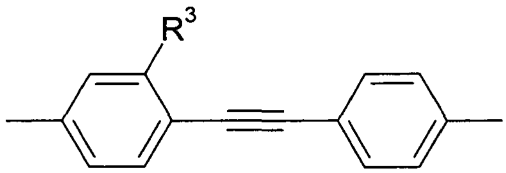
,
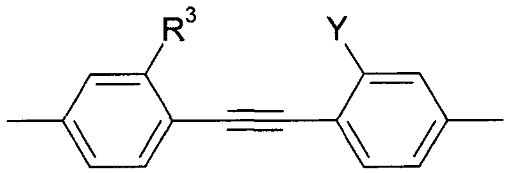
,
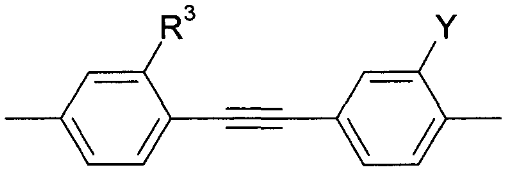
,
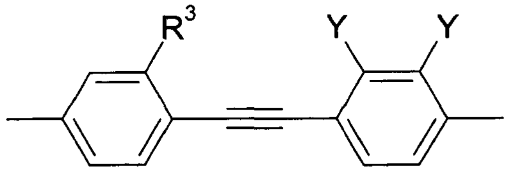
,
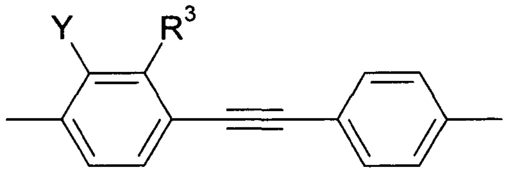
,
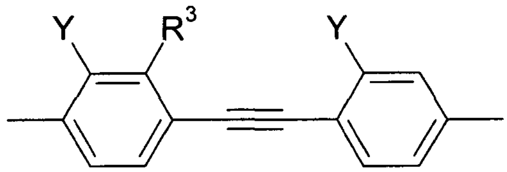
,
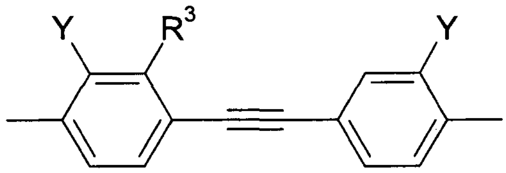
,
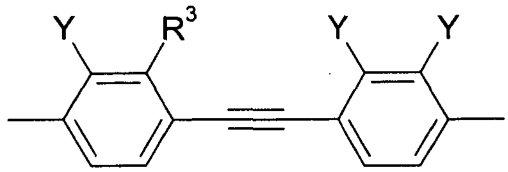
,
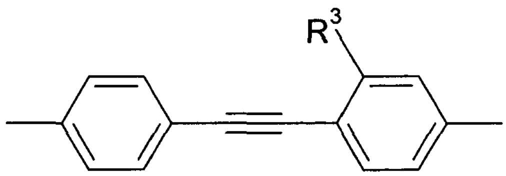
,
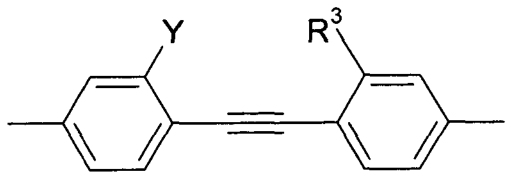
,
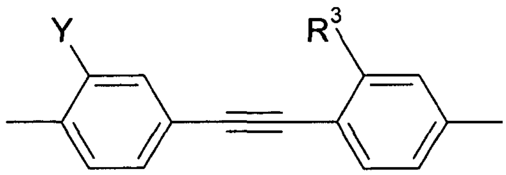
,
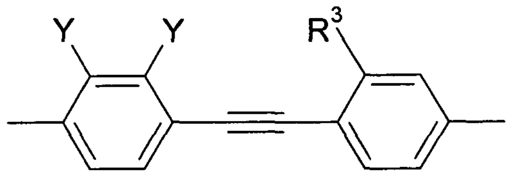
,

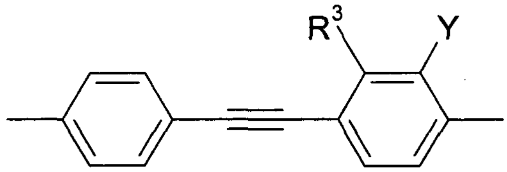
,
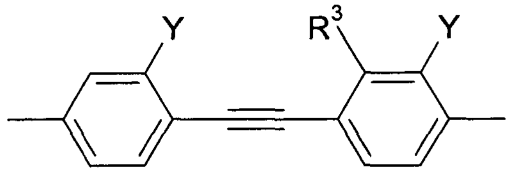
,

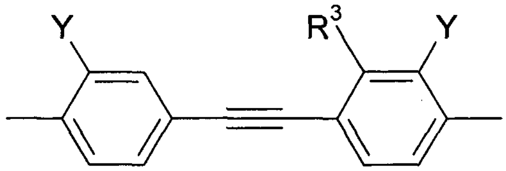

oder

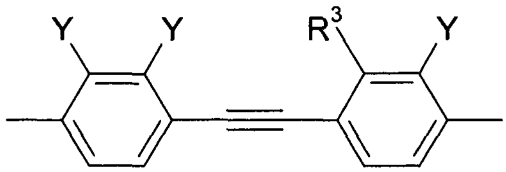

bedeuten.
worin die Parameter die in Anspruch 1 gegeben Bedeutungen haben.

3. Verbindungen nach Anspruch 1 oder 2, **dadurch gekennzeichnet, dass** (m + n + o) 2 ist.

4. Verbindungen nach mindestens einem der Ansprüche 1 bis 3, **dadurch gekennzeichnet, dass** die Ringe $A^1$ bis $A^5$, soweit vorhanden, je einen optional substituierten 1,4-Phenylenring bedeuten.

5. Verbindungen nach mindestens einem der Ansprüche 1 bis 4, **dadurch gekennzeichnet, dass** m 0 ist.

6. Verbindungen nach mindestens einem der Ansprüche 1 bis 5, ausgewählt aus der Gruppe der Verbindungen der Formeln

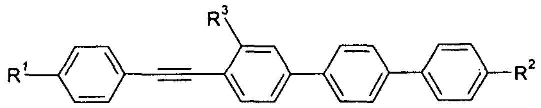

und

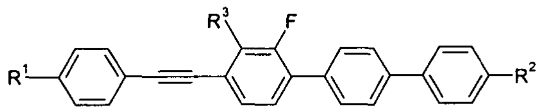

worin die Parameter die in Anspruch 1 gegeben Bedeutungen haben.

7. Flüssigkristallmedium, **dadurch gekennzeichnet, dass** es eine oder mehrere Verbindungen der Formel I nach mindestens einem der Ansprüche 1 bis 6 enthält.

8. Flüssigkristallmedium nach Anspruch 7, **dadurch gekennzeichnet, dass** es zusätzlich eine oder mehrere Verbindungen ausgewählt aus den Verbindungen der Formel II enthält:

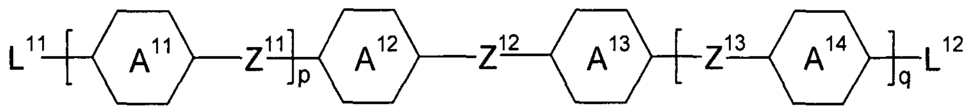 II

worin bedeuten:

$L^{11}$ $R^{11}$ oder $X^{11}$,
$L^{12}$ $R^{12}$ oder $X^{12}$,
$R^{11}$ und $R^{12}$ unabhängig voneinander unfluoriertes Alkyl oder unfluoriertes Alkoxy mit 1 bis 17 C-Atomen oder unfluoriertes Alkenyl, unfluoriertes Alkinyl, unfluoriertes Alkenyloxy, oder unfluoriertes Alkoxyalkyl mit 2 bis 15 C-Atomen,
$X^{11}$ und $X^{12}$ unabhängig voneinander F, Cl, Br, -CN, -NCS, -SCN, $-SF_5$, fluoriertes Alkyl oder fluoriertes Alkoxy mit 1 bis 7 C-Atomen oder fluoriertes Alkenyl, fluoriertes Alkenyloxy oder fluoriertes Alkoxyalkyl mit 2 bis 7 C-Atomen,
p, q unabhängig 0 oder 1,
$Z^{11}$ bis $Z^{13}$ unabhängig voneinander *trans*- -CH=CH-, *trans*- -CF=CF-, -C≡C- oder eine Einfachbindung, und

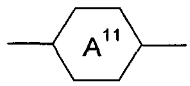 bis 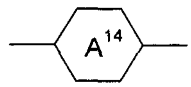

unabhängig voneinander

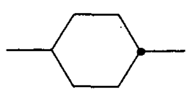, 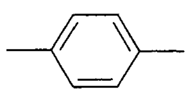, 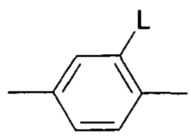,

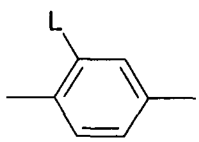, 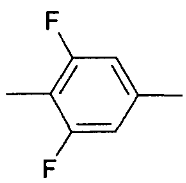, 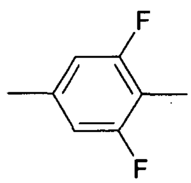,

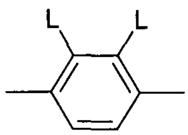,

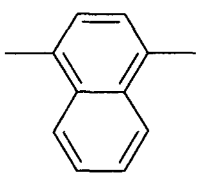, 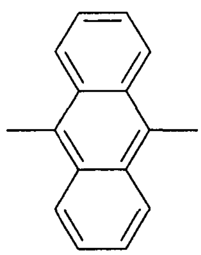 oder 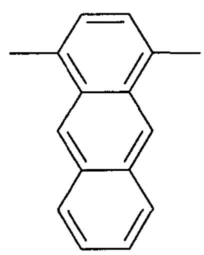,

worin L unabhängig verzweigtes oder unverzweigtes Alkyl, Alkenyl oder Alkinyl mit 1 bis 12 C-Atomen, worin unabhängig voneinander auch eine oder mehrere "$-CH_2-$"-Gruppen durch O ersetzt sein können, $C_3$-$C_6$ Cycloalkyl, $C_3$-$C_6$ Cycloalkenyl, fluoriertes Alkyl oder Alkenyl, fluoriertes Alkyloxy oder Alkenyloxy, F, Cl, Br, CN, NCS, SCN oder $SF_5$, bedeutet.

**9.** Flüssigkristallmedium nach Anspruch 7 oder 8, **dadurch gekennzeichnet, dass** die Konzentration der Verbindungen der Formel I im Medium im Bereich von insgesamt 5 % bis 95 % liegt.

**10.** Verwendung der Verbindungen der Formel I nach einem der Ansprüche 1 bis 6 in einem Bauteil für die Hochfrequenztechnik.

**11.** Verwendung der Verbindungen der Formel I nach einem der Ansprüche 1 bis 6 in einem flüssigkristallinen Medium.

**12.** Verfahren zur Herstellung eines Flüssigkristallmediums nach mindestens einem der Ansprüche 7 bis 9, **dadurch gekennzeichnet, dass** eine oder mehrere Verbindungen der Formel I mit einer oder mehreren weiteren Verbindungen und optional mit einem oder mehreren Additiven gemischt wird.

**13.** Bauteil für die Hochfrequenztechnik, **dadurch gekennzeichnet, dass** es ein Flüssigkristallmedium nach mindestens einem der Ansprüche 7 bis 9 enthält.

**14.** Bauteil nach Anspruch 13, **dadurch gekennzeichnet, dass** es sich um einen oder mehrere funktionell verbundene Phasenschieber handelt.

**15.** Verwendung eines Flüssigkristallmediums nach mindestens einem der Ansprüche 7 bis 9 in einem Bauteil für die Hochfrequenztechnik.

**16.** 'Phased array' Antenne, **dadurch gekennzeichnet**, das sie ein oder mehrere Bauteile nach Anspruch 13 oder 14 enthält.

**Claims**

**1.** Compounds of the formula I

$$R^1\text{-}[A^1\text{-}Z^1]_m\text{-}A^2 \equiv A^3\text{-}[Z^2\text{-}A^4]_n\text{-}[Z^3\text{-}A^5]_o\text{-}R^2 \qquad \text{I}$$

in which

one of $A^2$ and $A^3$ denotes

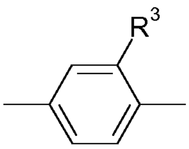
,
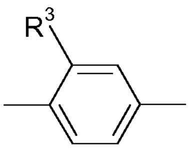
,
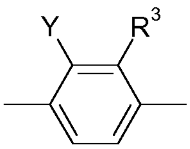
,
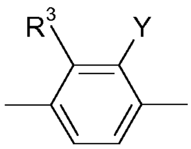

or

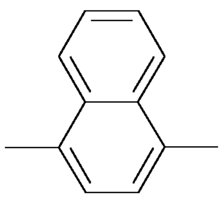
,

the other of $A^2$ and $A^3$, $A^1$, $A^4$ and $A^5$, independently of one another, denote

a) 1,4-phenylene, in which one or more CH groups may be replaced by N,
b) a radical of the formula

c) trans-1,4-cyclohexylene or cyclohexenylene, in which, in addition, one or two non-adjacent $CH_2$ groups may be replaced by -O-and/or -S-,

or

d) a radical from the group 1,4-bicyclo[2.2.2]octylene, cyclobutane-1,3-diyl, spiro[3.3]heptane-2,6-diyl, thiophene-2,5-diyl, furan-2,5-diyl,

and in which, in the groups a), b), c) and d),
one or more H atoms are optionally replaced, independently, by in each case one group Y,
Y denotes Br, Cl, F, CN, -NCS, -SCN, $SF_5$, $C_2$-$C_{10}$ alkenyl, $C_1$-$C_{10}$ alkoxy, $C_3$-$C_6$ cycloalkyl, $C_3$-$C_6$ cycloalkenyl or a mono- or polyfluorinated $C_1$-$C_{10}$ alkyl or alkoxy group,
$R^1$ and $R^2$ denote a straight-chain alkyl radical having 1 to 15 C atoms, where, in addition, one or more $CH_2$ groups in these radicals may each be replaced, independently of one another, by -C≡C-, -CH=CH-, -(CO)O-, -O(CO)-, -(CO)-, -O- in such a way that O atoms are not linked directly to one another,

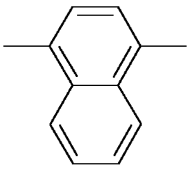
,
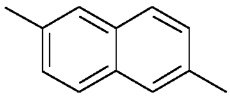
,
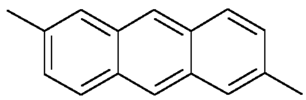
,

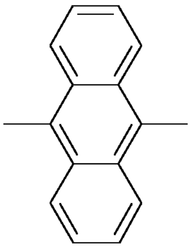
,
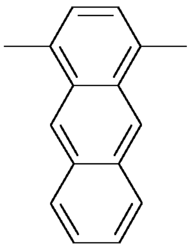
,
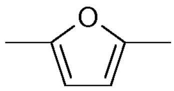
or
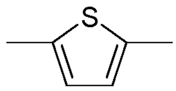
,

$R^3$ denotes $C_1$-$C_{10}$ alkyl, $C_2$-$C_{10}$ alkenyl, $C_1$-$C_{10}$ alkoxy, $C_3$-$C_6$ cycloalkyl, $C_3$-$C_6$ cycloalkenyl or a mono- or polyfluorinated $C_1$-$C_{10}$ alkyl or alkoxy group,
$Z^1$, $Z^2$ and $Z^3$ denote a single bond, and
m, n and o, independently of one another, denote 0 or 1, where (m + n + o) is 2 or 3.

2. Compounds according to Claim 1, **characterised in that** the sub-formula "-$A^2$-≡-$A^3$-" of the formula I is selected from the group of the following formulae:

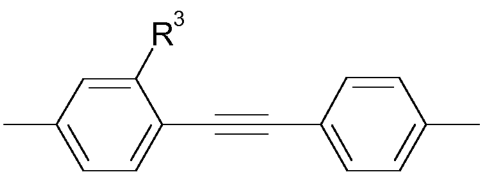
,
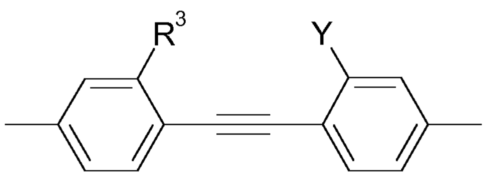
,

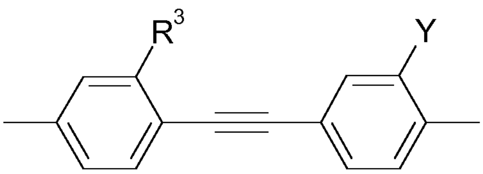
,
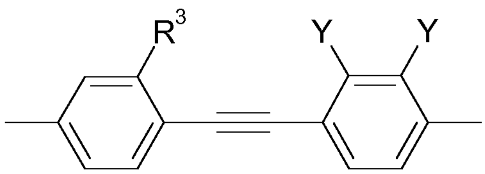
,

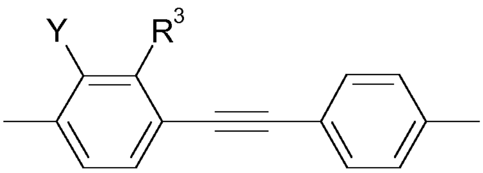,
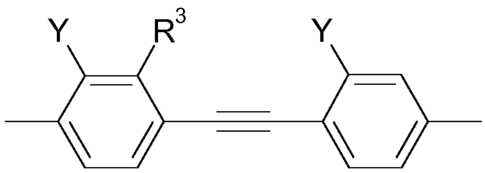,

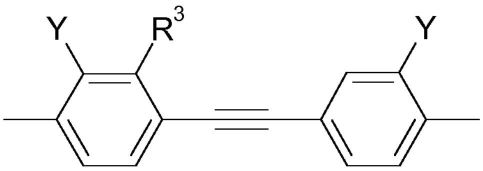,
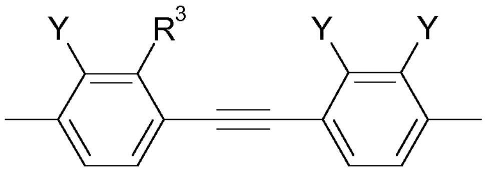,

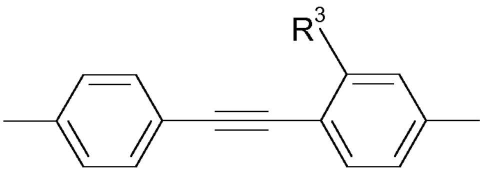,
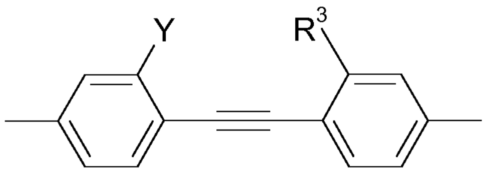,

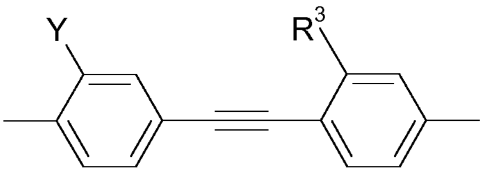,
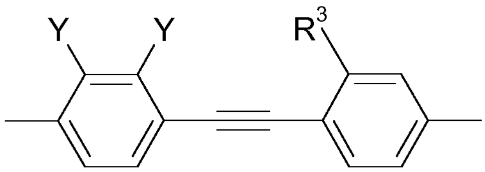,

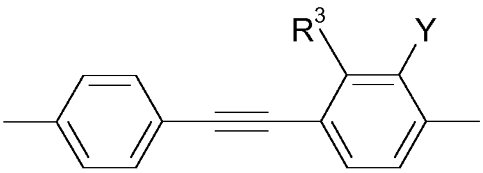,
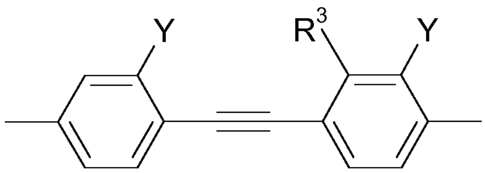,

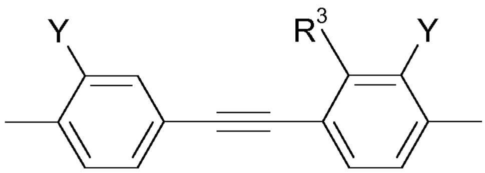

or

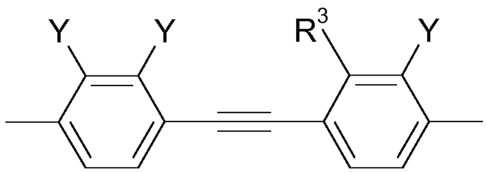

in which the parameters have the meanings given in Claim 1.

**3.** Compounds according to Claim 1 or 2, **characterised in that** (m + n + o) is 2.

**4.** Compounds according to at least one of Claims 1 to 3, **characterised in that** the rings $A^1$ to $A^5$, if present, each denote an optionally substituted 1,4-phenylene ring.

**5.** Compounds according to at least one of Claims 1 to 4, **characterised in that** m is 0.

**6.** Compounds according to at least one of Claims 1 to 5, selected from the group of the compounds of the formulae

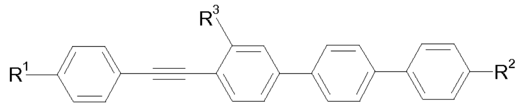

and

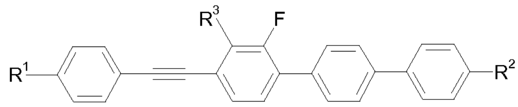

in which the parameters have the meanings given in Claim 1.

**7.** Liquid-crystal medium, **characterised in that** it comprises one or more compounds of the formula I according to at least one of Claims 1 to 6.

**8.** Liquid-crystal medium according to Claim 7, **characterised in that** it additionally comprises one or more compounds selected from the compounds of the formula II:

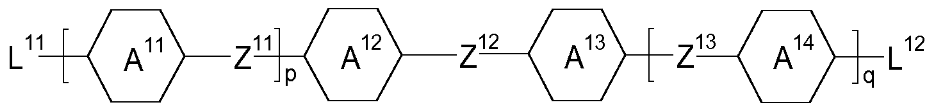

II

in which:

$L^{11}$ denotes $R^{11}$ or $X^{11}$,
$L^{12}$ denotes $R^{12}$ or $X^{12}$,
$R^{11}$ and $R^{12}$, independently of one another, denote unfluorinated alkyl or unfluorinated alkoxy having 1 to 17 C atoms or unfluorinated alkenyl, unfluorinated alkynyl, unfluorinated alkenyloxy or unfluorinated alkoxyalkyl having 2 to 15 C atoms,
$X^{11}$ and $X^{12}$, independently of one another, denote F, Cl, Br, -CN, -NCS, -SCN, $-SF_5$, fluorinated alkyl or fluorinated alkoxy having 1 to 7 C atoms or fluorinated alkenyl, fluorinated alkenyloxy or fluorinated alkoxyalkyl having 2 to 7 C atoms,
p, q independently denote 0 or 1,
$Z^{11}$ to $Z^{13}$, independently of one another, denote *trans* -CH=CH-, *trans* -CF=CF-, -C≡C- or a single bond, and

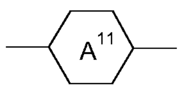

to

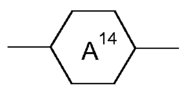

,

independently of one another, denote

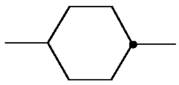 , 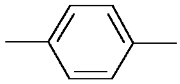 , 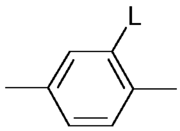 ,

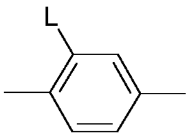 , 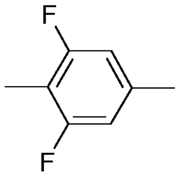 , 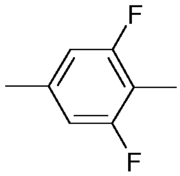 ,

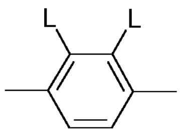 ,

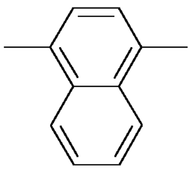 , 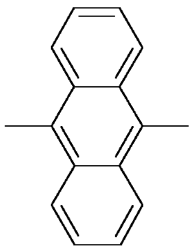 or 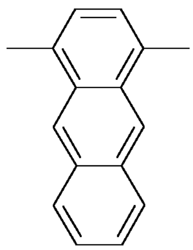 ,

in which L independently denotes branched or unbranched alkyl, alkenyl or alkynyl having 1 to 12 C atoms, in which, in addition, one or more "$-CH_2-$" groups may be replaced, independently of one another, by O, or denotes $C_3$-$C_6$ cycloalkyl, $C_3$-$C_6$ cycloalkenyl, fluorinated alkyl or alkenyl, fluorinated alkoxy or alkenyloxy, F, Cl, Br, CN, NCS, SCN or $SF_5$.

**9.** Liquid-crystal medium according to Claim 7 or 8, **characterised in that** the concentration of the compounds of the formula I in the medium is in the range from in total 5% to 95%.

**10.** Use of the compounds of the formula I according to one of Claims 1 to 6 in a component for high-frequency technology.

**11.** Use of the compounds of the formula I according to one of Claims 1 to 6 in a liquid-crystalline medium.

**12.** Process for the preparation of a liquid-crystal medium according to at least one of Claims 7 to 9, **characterised in that** one or more compounds of the formula I are mixed with one or more further compounds and optionally with one or more additives.

**13.** Component for high-frequency technology, **characterised in that** it contains a liquid-crystal medium according to at least one of Claims 7 to 9.

**14.** Component according to Claim 13, **characterised in that** it is one or more functionally connected phase shifters.

**15.** Use of a liquid-crystal medium according to at least one of Claims 7 to 9 in a component for high-frequency technology.

**16.** Phased array antenna, **characterised in that** it comprises one or more components according to Claim 13 or 14.

## Revendications

**1.** Composés de la formule I :

$$R^1-[A^1-Z^1]_m-A^2 \equiv A^3-[Z^2-A^4]_n-[Z^3-A^5]_o-R^2 \qquad I$$

dans laquelle :

l'un de $A^2$ et $A^3$ représente :

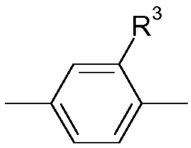,
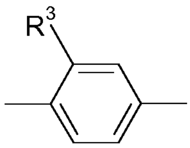,
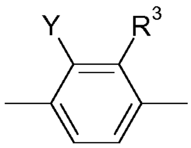,
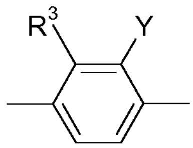

ou

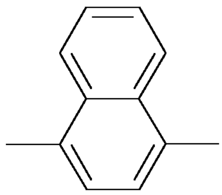,

l'autre de $A^2$ et $A^3$, $A^1$, $A^4$ et $A^5$ représentent, de manière indépendante les uns des autres :

a) 1,4-phénylène, où un ou plusieurs groupe(s) CH peut/ peuvent être remplacé(s) par N ;
b) un radical de la formule :

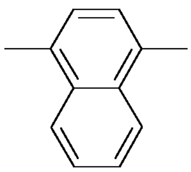,
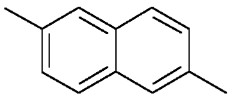,
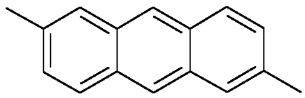,

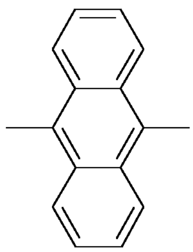,
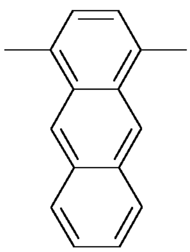,
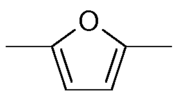
ou
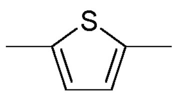,

c) trans-1,4-cyclohexylène ou cyclohexénylène, où, en outre, un ou deux groupe(s) $CH_2$ non adjacents peut/peuvent être remplacé(s) par -O- et/ou -S- ;
ou
d) un radical pris parmi le groupe constitué par 1,4-bicyclo-[2.2.2]octylène, cyclobutane-1,3-diyle, spiro[3.3]heptane-2,6-diyle, thiophène-2,5-diyle, furan-2,5-diyle ;

et où, dans les groupes a), b), c) et d),
un ou plusieurs atome(s) de H est/sont en option remplacé(s), de manière indépendante, par, dans chaque cas, un groupe Y ;
Y représente Br, Cl, F, CN, -NCS, -SCN, $SF_5$, $C_2$-$C_{10}$ alkényle, $C_1$-$C_{10}$ alcoxy, $C_3$-$C_6$ cycloalkyle, $C_3$-$C_6$ cycloalkényle ou un groupe $C_1$-$C_{10}$ alkyle ou alcoxy mono- ou polyfluoré ;
$R^1$ et $R^2$ représentent un radical alkyle en chaîne droite qui comporte de 1 à 15 atome(s) de C, où, en outre,

un ou plusieurs groupe(s) $CH_2$ dans ces radicaux peut/peuvent chacun être remplacé(s), de manière indépendante les uns des autres, par -C≡C-, -CH=CH-, -(CO)O-, -O(CO)-, -(CO)-, -O- de telle sorte que des atomes de O ne soient pas liés directement les uns aux autres ;

$R^3$ représente $C_1$-$C_{10}$ alkyle, $C_2$-$C_{10}$ alkényle, $C_1$-$C_{10}$ alcoxy, $C_3$-$C_6$ cycloalkyle, $C_3$-$C_6$ cycloalkényle ou un groupe $C_1$-$C_{10}$ alkyle ou alcoxy mono- ou polyfluoré ;

$Z^1$, $Z^2$ et $Z^3$ représentent une liaison simple ; et

m, n et o représentent, de manière indépendante les uns des autres, 0 ou 1, où (m + n + o) est 2 ou 3.

2. Composés selon la revendication 1, **caractérisés en ce que** la sous-formule "-$A^2$-≡-$A^3$-" de la formule I est sélectionnée parmi le groupe des formules qui suivent :

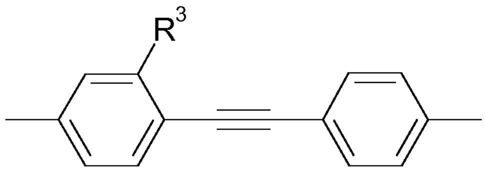
,
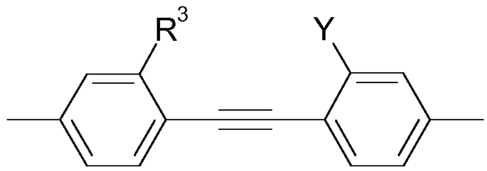
,
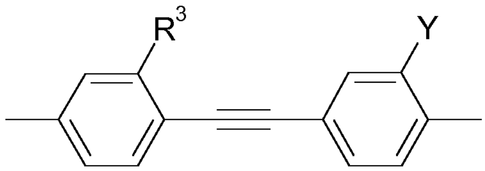
,
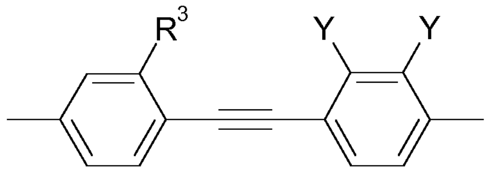
,
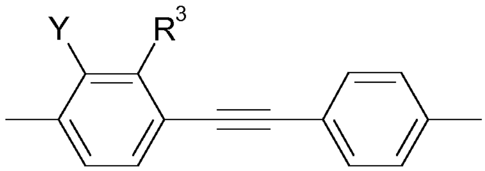
,
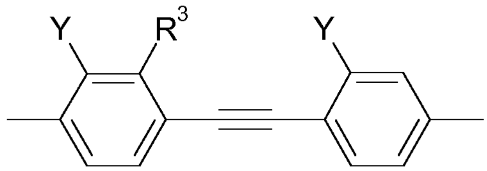
,
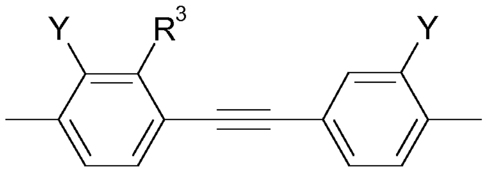
,
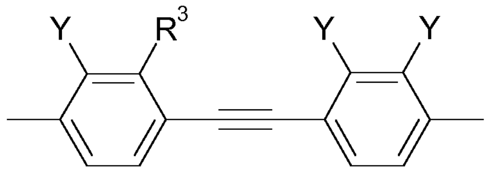
,
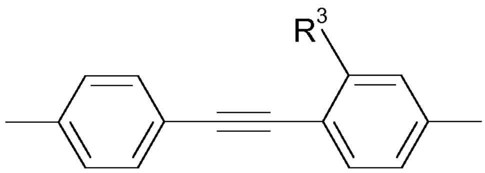
,
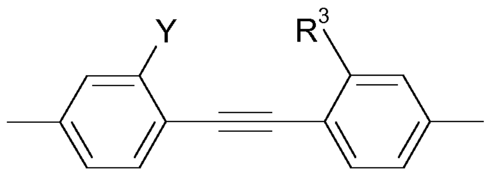
,
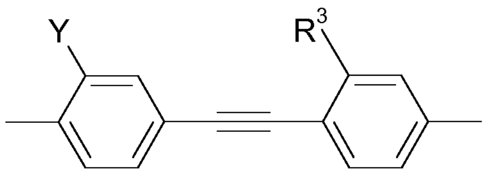
,
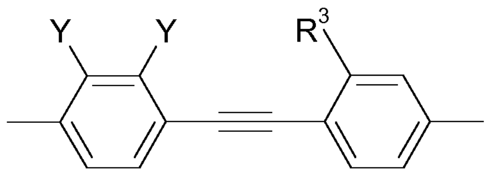
,

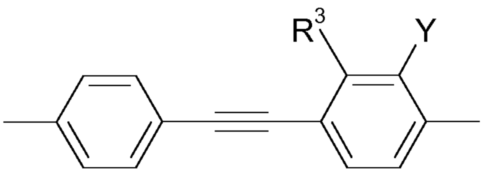

,

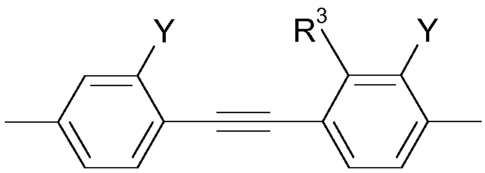

,

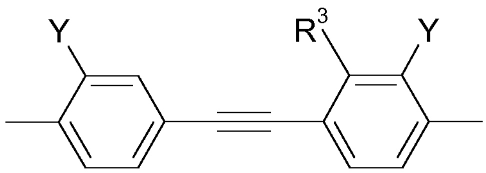

ou

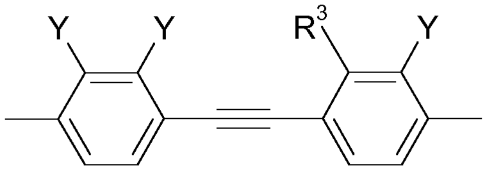

dans lesquelles les paramètres présentent les significations qui ont été données selon la revendication 1.

**3.** Composés selon la revendication 1 ou 2, **caractérisés en ce que** (m + n + o) est 2.

**4.** Composés selon au moins l'une des revendications 1 à 3, **caractérisés en ce que** les cycles $A^1$ à $A^5$, s'ils sont présents, représentent chacun un cycle 1,4-phénylène en option substitué.

**5.** Composés selon au moins l'une des revendications 1 à 4, **caractérisés en ce que** m est 0.

**6.** Composés selon au moins l'une des revendications 1 à 5, sélectionnés parmi le groupe des composés des formules :

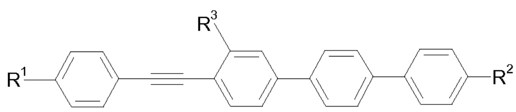

et

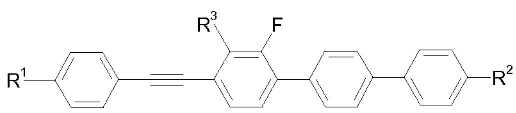

dans lesquelles les paramètres présentent les significations qui ont été données selon la revendication 1.

**7.** Milieu cristallin liquide, **caractérisé en ce qu'**il comprend un ou plusieurs composé(s) de la formule I selon au moins l'une des revendications 1 à 6.

**8.** Milieu cristallin liquide selon la revendication 7, **caractérisé en ce qu'**il comprend de façon additionnelle un ou plusieurs composé(s) qui est/sont sélectionné(s) parmi les composés de la formule II :

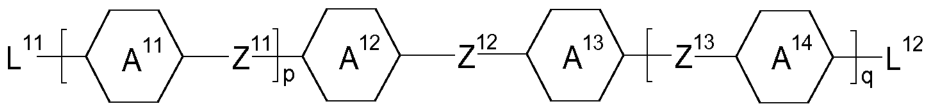 II

dans laquelle :

$L^{11}$ représente $R^{11}$ ou $X^{11}$ ;
$L^{12}$ représente $R^{12}$ ou $X^{12}$ ;
$R^{11}$ et $R^{12}$ représentent, de manière indépendante l'un de l'autre, alkyle non fluoré ou alcoxy non fluoré qui comporte de 1 à 17 atome(s) de C ou alkényle non fluoré, alkynyle non fluoré, alkényloxy non fluoré ou alcoxyalkyle non fluoré qui comporte de 2 à 15 atomes de C ;
$X^{11}$ et $X^{12}$ représentent, de manière indépendante l'un de l'autre, F, Cl, Br, -CN, -NCS, -SCN, $-SF_5$, alkyle fluoré ou alcoxy fluoré qui comporte de 1 à 7 atome(s) de C ou alkényle fluoré, alkényloxy fluoré ou alcoxyalkyle fluoré qui comporte de 2 à 7 atomes de C ;
p, q représentent de manière indépendante 0 ou 1 ;
$Z^{11}$ à $Z^{13}$ représentent, de manière indépendante les uns des autres, *trans* -CH=CH-, *trans* -CF=CF-, -C≡C- ou une liaison simple ; et

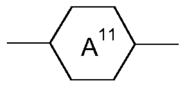

à

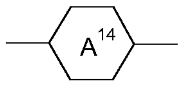

,

représentent, de manière indépendante les uns des autres,

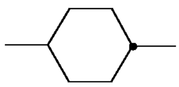 , 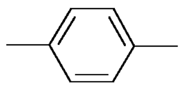 , 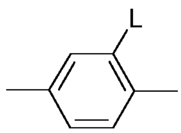 ,

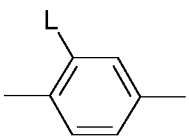 , 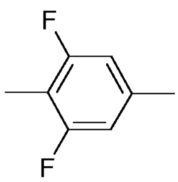 , 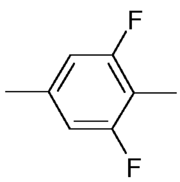 ,

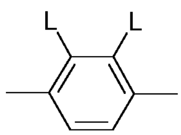 ,

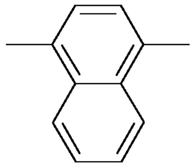 , 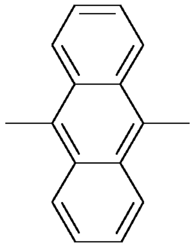 ou 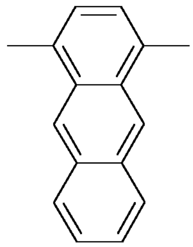 ,

où L représente de manière indépendante alkyle, alkényle ou alkynyle ramifié ou non ramifié qui comporte de 1 à 12 atome(s) de C, où, en outre, un ou plusieurs groupe(s) "-$CH_2$-" peut/peuvent être remplacé(s), de manière indépendante les uns des autres, par O, ou représente $C_3$-$C_6$ cycloalkyle, $C_3$-$C_6$ cycloalkényle, alkyle ou alkényle fluoré, alcoxy ou alkényloxy fluoré, F, Cl, Br, CN, NCS, SCN ou $SF_5$.

**9.** Milieu cristallin liquide selon la revendication 7 ou 8, **caractérisé en ce que** la concentration des composés de la formule I dans le milieu s'inscrit à l'intérieur de la plage qui va au total de 5 % à 95 %.

**10.** Utilisation des composés de la formule I selon l'une des revendications 1 à 6 dans un composant pour la technologie des hautes fréquences.

**11.** Utilisation des composés de la formule I selon l'une des revendications 1 à 6 dans un milieu cristallin liquide.

**12.** Procédé pour la préparation d'un milieu cristallin liquide selon au moins l'une des revendications 7 à 9, **caractérisé en ce qu'**un ou plusieurs composé(s) de la formule I est/sont mélangé(s) avec un ou plusieurs autre(s) composé(s) et, en option, avec un ou plusieurs additif(s).

**13.** Composant pour la technologie des hautes fréquences, **caractérisé en ce qu'**il contient un milieu cristallin liquide selon au moins l'une des revendications 7 à 9.

**14.** Composant selon la revendication 13, **caractérisé en ce qu'**il s'agit d'un ou de plusieurs déphaseur(s) connectés fonctionnellement.

**15.** Utilisation d'un milieu cristallin liquide selon au moins l'une des revendications 7 à 9 dans un composant pour la technologie des hautes fréquences.

**16.** Antenne en réseau à commande de phase, **caractérisée en ce qu'**elle comprend un ou plusieurs composant(s) selon la revendication 13 ou 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102004029429 A
- JP 2005120208 A
- JP 5255151 A
- WO 2009125721 A1
- JP 10045642 A
- DE 10120024
- WO 2009125721 A
- DE 102012003876
- WO 2012097853 A1
- DE 1020111112950 A1
- WO 2012095139 A1


### In der Beschreibung aufgeführte Nicht-Patentliteratur


- **A. GAEBLER ; A. MOESSINGER ; F. GOELDEN et al.** Liquid Crystal-Reconfigurable Antenna Concepts for Space Applications at Microwave and Millimeter Waves. *International Journal of Antennas and Propagation,* 2009, vol. 2009, 7
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35 GHz. *34th European Microwave Conference,* 545-548
- **SHIN-TSON WU et al.** *Jpn. J. Appl. Phys.,* 1999, vol. 38, 286-288
- **SHIN-TSON WU et al.** *Jpn. J. Appl. Phys.,* 2000, vol. 39, 38-41
- Merck Liquid Crystals, Physical Properties of Liquid Crystals. Merck KGaA, November 1997
- **A. PENIRSCHKE et al.** Cavity Perturbation Method for Characterization of Liquid Crystals up to 35GHz. *34th European Microwave Conference,* 545-548
- Direct Simulation of Material Permittivities ... **A. GAEBLER et al.** 12MTC 2009 - International Instrumentation and Measurement Technology Conference. IEEE, 2009, 463-467
- **A. PENIRSCHKE et al.** *34th European Microwave Conference,* 545-548
- *CHEMICAL ABSTRACTS,* 1073-06-9
- *CHEMICAL ABSTRACTS,* 145413-17-8
- *CHEMICAL ABSTRACTS,* 1375922-99-8